# EUROPÄISCHE PATENTSCHRIFT

(11) **EP 1 984 324 B1**
(45) Veröffentlichungstag und Bekanntmachung des Hinweises auf die Patenterteilung: **18.05.2011**
(21) Anmeldenummer: 07702867.8
(22) Anmeldetag: 18.01.2007
(51) Int. Cl.: C07C 243/38, A61K 31/15, A61P 3/00

(54) **MANDELSÄUREHYDRAZIDE**
MANDELIC HYDRAZIDES
HYDRAZIDES D'ACIDE MANDÉLIQUE

(30) Priorität: 14.02.2006 DE 102006006648
(43) Veröffentlichungstag der Anmeldung: 29.10.2008
(73) Patentinhaber: Merck Patent GmbH, 64293 Darmstadt (DE)
(72) Erfinder: GERICKE, Rolf, 64342 Seeheim-Jugenheim (DE); KLEIN, Markus, 64291 Damstadt (DE); MEDERSKI, Werner, 64673 Zwingenberg (DE); BEIER, Norbert, 64354 Reinheim (DE); LANG, Florian, 72076 Tuebingen (DE)
(86) Internationale Anmeldenummer: PCT/EP2007/000430
(87) Internationale Veröffentlichungsnummer: WO 2007/093264

(56) Entgegenhaltungen:
- WO-A-2005/037773
- WO-A-2006/105850

## Beschreibung

Der Erfindung lag die Aufgabe zugrunde, neue Verbindungen mit wertvollen Eigenschaften aufzufinden, insbesondere solche, die zur Herstellung von Arzneimitteln verwendet werden können.

Die vorliegende Erfindung betrifft Verbindungen, bei denen die Hemmung, Regulierung und/oder Modulation der Signaltransduktion von Kinasen, insbesondere der zellvolumenregulierten humanen Kinase h-sgk (human serum and glucocorticoid dependent kinase oder SGK) eine Rolle spielt, ferner pharmazeutische Zusammensetzungen, die diese Verbindungen enthalten, sowie die Verwendung der Verbindungen zur Behandlung SGKbedingter Krankheiten.

Die SGK mit den Isoformen SGK-1, SGK-2 und SGK-3 sind eine Serin/Threonin-Proteinkinase Familie (WO 02/17893).

Die erfindungsgemäßen Verbindungen sind vorzugsweise selektive Inhibitoren der SGK-1. Ferner können sie Inhibitoren der SGK-2 und/oder SGK-3 sein.

Im einzelnen betrifft die vorliegende Erfindung Verbindungen, die die Signaltransduktion der SGK hemmen, regulieren und/oder modulieren, Zusammensetzungen, die diese Verbindungen enthalten, sowie Verfahren zu ihrer Verwendung zur Behandlung von SGK-bedingten Krankheiten und Leiden wie Diabetes (z.B. Diabetes mellitus, diabetische Nephropathie, diabetische Neuropathie, diabetische Angiopathie und Mikroangiopathie), Fettsucht, metabolisches Syndrom (Dyslipidämie), systemische und pulmonale Hypertonie, Herzkreislauferkrankungen (z.B. kardiale Fibrosen nach Myokardinfarkt, Herzhypertrophie und Herzinsuffizienz, Arteriosklerose) und Nierenerkrankungen (z.B. Glomerulosklerose, Nephrosklerose, Nephritis, Nephropathie, Störung der Elektrolytausscheidung), allgemein bei jeglicher Art von Fibrosen und entzündlichen Prozessen (z.B. Leberzirrhose, Lungenfibrose, fibrosierende Pankreatitis, Rheumatismus und Arthrosen, Morbus Crohn, chronische Bronchitis, Strahlenfibrose, Sklerodermitis, zystische Fibrose, Narbenbildung, Morbus Alzheimer).

Die erfindungsgemäßen Verbindungen können auch das Wachstum von Tumorzellen und Tumormetastasen hemmen und sind deshalb für die Tumortherapie geeignet.

Die erfindungsgemäßen Verbindungen finden auch Verwendung bei der Behandlung von peptischen Ulcera, insbesondere bei Formen, die durch Stress ausgelöst werden.

Die erfindungsgemäßen Verbindungen finden weiterhin Verwendung zur Behandlung von Koagulopathien, wie z.B. Dysfibrinogenämie, Hypoprokonvertinämie, Hämophilie B, Stuart-Prower-Defekt, ProthrombinKomplex-Mangel, Verbrauchskoagulopathie, Hyperfibrinolyse, Immunokoagulopathie oder komplexer Koagulopathien, wie auch bei neuronaler Erregbarkeit, z.B. Epilepsie. Die erfindungsgemäßen Verbindungen können auch bei der Behandlung eines Glaukoms oder Katarakt therapeutisch eingesetzt werden.

Die erfindungsgemäßen Verbindungen finden ferner Verwendung bei der Behandlung bakterieller Infektionen sowie in einer antiinfektiösen Therapie. Die erfindungsgemäßen Verbindungen können auch zur Steigerung der Lernfähigkeit und Aufmerksamkeit therapeutisch eingesetzt werden. Darüberhinaus wirken die erfindungsgemäßen Verbindungen der Zellalterung und Stress entgegen und steigern somit die Lebenserwartung und die Fitness im Alter.

Die erfindungsgemäßen Verbindungen finden ferner Verwendung bei der Behandlung von Tinitus.

Die Identifikation von kleinen Verbindungen, die die Signaltransduktion der SGK spezifisch hemmen, regulieren und/oder modulieren, ist daher wünschenswert und ein Ziel der vorliegenden Erfindung.

Es wurde gefunden, daß die erfindungsgemäßen Verbindungen und ihre Salze bei guter Verträglichkeit sehr wertvolle pharmakologische Eigenschaften besitzen.

Insbesondere zeigen sie inhibierende Eigenschaften der SGK.

Gegenstand der vorliegenden Erfindung sind deshalb erfindungsgemäße Verbindungen als Arzneimittel und/oder Arzneimittelwirkstoffe bei der Behandlung und/oder Prophylaxe der genannten Erkrankungen und die Verwendung von erfindungsgemäßen Verbindungen zur Herstellung eines Pharmazeutikums für die Behandlung und/oder Prophylaxe der genannten Erkrankungen wie auch ein Verfahren zur Behandlung der genannten Erkrankungen umfassend die Verabreichung eines oder mehrerer erfindungsgemäßer Verbindungen an einen Patienten mit Bedarf an einer derartigen Verabreichung.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Zur Identifizierung eines Signalübertragungswegs und zum Nachweis von Wechselwirkungen zwischen verschiedenen Signalübertragungswegen wurden von verschiedenen Wissenschaftlern geeignete Modelle oder Modellsysteme entwickelt, z.B. Zellkulturmodelle (z.B. Khwaja et al., EMBO, 1997, 16; 2783-93) und Modelle transgener Tiere (z.B. White et al., Oncogene, 2001, 20, 7064-7072). Zur Bestimmung bestimmter Stufen in der Signalübertragungskaskade können wechselwirkende Verbindungen genutzt werden, um das Signal zu modulieren (z.B. Stephens et al., Biochemical J., 2000, 351, 95-105). Die erfindungsgemäßen Verbindungen können auch als Reagenzien zur Testung kinaseabhängiger Signalübertragungswege in Tieren und/oder Zellkulturmodellen oder in den in dieser Anmeldung genannten klinischen Erkrankungen verwendet werden.

Die Messung der Kinaseaktivität ist eine dem Fachmann wohlbekannt Technik. Generische Testsysteme zur Bestimmung der Kinaseaktivität mit Substraten, z.B. Histon (z.B. Alessi et al., FEBS Lett. 1996, 399, 3, Seiten 333-338) oder dem basischen Myelinprotein sind in der Literatur beschrieben (z.B. Campos-Gonzälez, R. und Glenney, Jr., J.R. 1992, J. Biol. Chem. 267, Seite 14535).

Zur Identifikation von Kinase-Inhibitoren stehen verschiedene Assay-Systeme zur Verfügung. Beim Scintillation-Proximity-Assay (Sorg et al., J. of. Biomolecular Screening, 2002, 7, 11-19) und dem FlashPlate-Assay wird die radioaktive Phosphorylierung eines Proteins oder Peptids als Substrat mit γATP gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer- (HTR-FRET-) und Fluoreszenzpolarisations- (FP-) Technologien als Assay-Verfahren nützlich (Sills et al., J. of Biomolecular Screening, 2002, 191-214).

Andere nicht radioaktive ELISA-Assay-Verfahren verwenden spezifische Phospho-Antikörper (Phospho-AK). Der Phospho-AK bindet nur das phosphorylierte Substrat. Diese Bindung ist mit einem zweiten Peroxidasekonjugierten Anti-Schaf-Antikörper durch Chemolumineszenz nachweisbar (Ross et al., Biochem. J., 2002, 366, 977-981).

### STAND DER TECHNIK

In der WO 00/62781 ist die Verwendung von Arzneimitteln enthaltend Hemmstoffe der zellvolumenregulierten humanen Kinase H-SGK beschrieben.

Acylhydrazone sind als SGK-Inhibitoren in der WO 2005037773 beschrieben. Acylhydraziole sind als SGK-Inhibitoren in der WO 2006 105850 beschrieben.

Andere Acylmandelsäurehydrazide sind als Fungizide in WO 96/17840 und von P. Legrel in Tetrahedron 1988, 44, 4805-4814 beschrieben. Benzyliden-benzohydrazide mit antibakterieller Wirkung sind in der WO 02/070464 A2 beschrieben. Die Verwendung von Acylhydraziden zur Behandlung bakterieller Infektionen ist in WO 01/70213 offenbart. Andere Acylhydrazonderivate, u.a. zur Behandlung von Diabeteskomplikationen, sind in JP 11-106371 offenbart.

Methoxysubstituierte aromatische Acylhydrazonderivate zur Behandlung von Krebs sind von T.Kametani et al. in Yakugaku Zasshi (1963), 83, 851-855 und in Yakugaku Zasshi (1963), 83, 844-847 beschrieben.

Andere aromatische Acylhydrazonderivate als Verstärker von Sedativa und zur Blutdrucksenkung sind in JP 41-20699 offenbart.

Die Verwendung von Kinase-Inhibitoren in der antiinfektiösen Therapie ist von C.Doerig in Cell. Mol. Biol. Lett. Vol.8, No. 2A, 2003, 524-525 beschrieben.

Die Verwendung von Kinase-Inhibitoren bei Fettsucht ist von N.Perrotti in J. Biol. Chem. 2001, März 23; 276(12):9406-9412 beschrieben.

In nachstehenden Literaturstellen wird die Verwendung von SGK-Hemmern bei der Behandlung von Krankheiten nahegelegt und/oder beschrieben:
1: Chung EJ, Sung YK, Farooq M, Kim Y, Im S, Tak WY, Hwang YJ, Kim YI, Han HS, Kim JC, Kim MK. Gene expression profile analysis in human hepatocellular carcinoma by cDNA microarray. Mol Cells. 2002; 14:382-7.
2: Brickley DR, Mikosz CA, Hagan CR, Conzen SD. Ubiquitin modification of serum and glucocorticoid-induced protein kinase-1(SGK-1). J Biol Chem. 2002;277:43064-70.
3: Fillon S, Klingel K, Wamtges S, Sauter M, Gabrysch S, Pestel S, Tanneur V, Waldegger S, Zipfel A, Viebahn R, Haussinger D, Broer S, Kandolf R, Lang F. Expression of the serine/threonine kinase hSGK1 in chronic viral hepatitis. Cell Physiol Biochem. 2002;12:47-54.
4: Brunet A, Park J, Tran H, Hu LS, Hemmings BA, Greenberg ME. Protein kinase SGK mediates survival signals by phosphorylating the forkhead transcription factor FKHRL1 (FOXO3a). Mol Cell Biol 2001;21:952-65
5: Mikosz CA, Brickley DR, Sharkey MS, Moran TW, Conzen SD. Glucocorticoid receptor-mediated protection from apoptosis is associated with induction of the serine/threonine survival kinase gene, sgk-1. J Biol Chem. 2001;276:16649-54.
6: Zuo Z, Urban G, Scammell JG, Dean NM, McLean TK, Aragon 1, Honkanen RE. Ser/Thr protein phosphatase type 5 (PP5) is a negative regulator of glucocorticoid receptor-mediated growth arrest. Biochemistry. 1999;38:8849-57.
7: Buse P, Tran SH, Luther E, Phu PT, Aponte GW, Firestone GL. Cell cycle and hormonal control of nuclear-cytoplasmic localization of the serum- and glucocorticoid-inducible protein kinase, Sgk, in mammary tumor cells. A novel convergence point of anti-proliferative and proliferative cell signalling pathways. J Biol Chem. 1999;274:7253-63.
8: M. Hertweck, C. Göbel, R. Baumeister: C.elegans SGK-1 is the critical component in the Akt/PKB Kinase complex to control stress response and life span. Developmental Cell, Vol. 6, 577-588, April, 2004.

### ZUSAMMENFASSUNG DER ERFINDUNG

Die Erfindung betrifft Verbindungen der Formel I 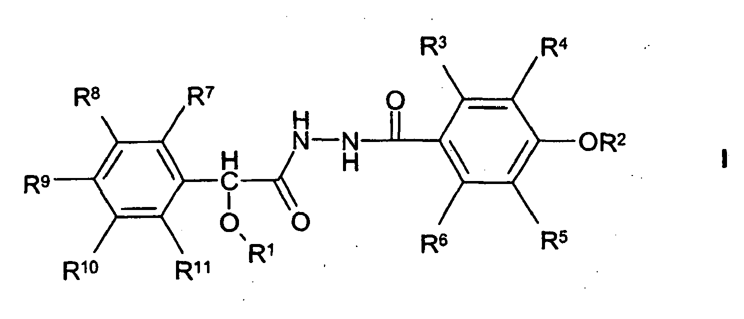 worin
R¹, R² jeweils unabhängig voneinander H, CHO oder Acetyl,

R³, R⁴, R⁵, R⁶, R⁷, R⁸, R⁹, R¹⁰, R¹¹ jeweils unabhängig voneinander
H, A, OSO₂A, Hal, NO₂, OR¹², N(R¹²)₂, CN, O-COA,
-[C(R¹²)₂]ₙCOOR¹², O-[C(R¹²)₂]ₒCOOR¹², SO₃H,
-[C(R¹²)₂]ₙAr, -CO-Ar, O-[C(R¹²)₂]ₙAr, -[C(R¹²)₂]ₙHet,
-[C(R¹²)₂]ₙC≡CH, O-[C(R¹²)₂]ₙC≡CH, -[C(R¹²)₂]ₙCON(R¹²)₂,
-[C(R¹²)₂]ₙCONR¹²N(R¹²)_{2,} O-[C(R¹²)₂]ₙCON(R¹²)₂,
O-[C(R¹²)₂]ₒCONR¹²N(R¹²)₂, NR¹²COA, NR¹²CON(R¹²)₂,
NR¹²SO₂A, N(SO₂A)₂, COR¹², S(O)ₘAr, SO₂NR¹² oder S(O)ₘA,
R³ und R⁴ zusammen auch CH=CH-CH=CH,
R³ und R⁴, R⁷ und R⁸
oder R⁸ und R⁹ zusammen auch Alkylen mit 3, 4 oder 5 C-Atomen,
worin eine oder zwei CH₂-Gruppen durch Sauerstoff ersetzt sein können,
A unverzweigtes oder verzweigtes Alkyl mit 1-6 C-Atomen, worin 1-7 H-Atome durch F ersetzt sein können, oder cylisches Alkyl mit 3-7 C-Atomen,
Ar unsubstituiertes oder ein-, zwei- oder dreifach durch Hal, A, OR¹², N(R¹² )₂, NO₂, CN, Phenyl, CON(R¹²)₂, NR¹²COA, NR¹²CON(R¹²)₂, NR¹²SO₂A, COR¹², SO₂N(R¹²)₂, S(O)ₘA, -[C(R¹²)₂]ₙ-COOR¹² und/oder -O[C(R¹²)₂]ₒ-COOR¹² substituiertes Phenyl, Naphthyl oder Biphenyl,
Het einen ein- oder zweikernigen gesättigten, ungesättigten oder aromatischen Heterocyclus mit 1 bis 4 N-, O- und/oder S-Atomen, der ein-, zwei- oder dreifach durch Hal, A, OR¹², N(R¹²)₂, NO₂, CN, COOR¹², CON(R¹²)₂, NR¹²COA, NR¹²SO₂A, COR¹², SO₂NR¹², S(O)ₘA, =S, =NR¹² und/oder =O (Carbonylsauerstoff) substituiert sein kann,
R¹² H oder A,
Hal F, Cl, Br oder I,
m 0, 1 oder 2,
n 0, 1, 2 oder 3,
o 1, 2 oder 3
bedeuten,
sowie ihre pharmazeutisch verwendbaren Salze, Solvate und Stereoisomere, einschließlich deren Mischungen in allen Verhältnissen.

Gegenstand der Erfindung sind die Verbindungen der Formel 1 und ihre Salze sowie ein Verfahren zur Herstellung von Verbindungen der Formel I nach den Ansprüchen 1-16 sowie ihrer pharmazeutisch verwendbaren Solvate, Salze und Stereoisomere, dadurch gekennzeichnet, daß man
a) eine Verbindung der Formel II 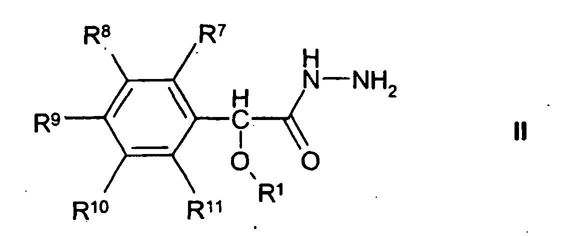 worin
   R¹, R⁷, R⁸, R⁹, R¹⁰ und R¹¹ die in Anspruch 1 angegebenen Bedeutungen haben,
   mit einer Verbindung der Formel III 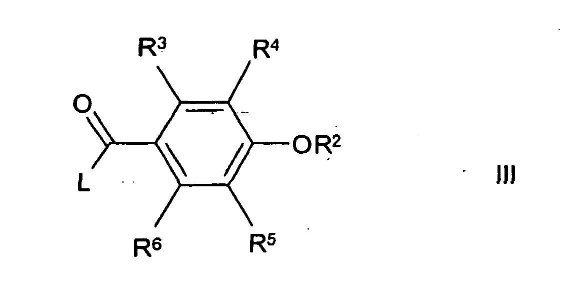 worin
   L Cl, Br, I oder eine freie oder reaktionsfähig funktionell
   abgewandelte OH-Gruppe bedeutet und
   R², R³, R⁴, R⁵ und R⁶ die in Anspruch 1 angegebenen
   Bedeutungen haben,
   umsetzt,
   oder
b) eine Verbindung der Formel IV 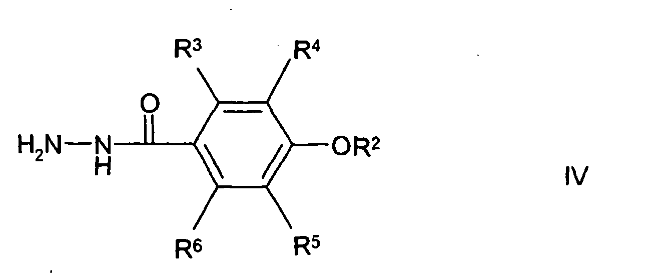 worin
   R² R³, R⁴, R⁵ und R⁶ die in Anspruch 1 angegebenen Bedeutungen haben,
   mit einer Verbindung der Formel V 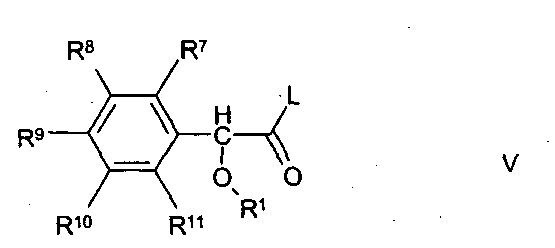 worin
   L Cl, Br, I oder eine freie oder reaktionsfähig funktionell
   abgewandelte OH-Gruppe bedeutet und
   R¹, R⁷, R⁸, R⁹, R¹⁰ und R¹¹ die in Anspruch 1 angegebenen
   Bedeutungen haben,
   umsetzt,
   oder
c) in einer Verbindung der Formel I einen Rest R¹, R², R³, R⁴, R⁵, R⁶, R⁷, R⁸, R⁹, R¹⁰ und/oder R¹¹ in einen anderen Rest R¹, R², R³, R⁴, R⁵, R⁶, R⁷, R⁸, R⁹, R¹⁰ und/oder R¹¹ umwandelt, indem man einen Ether durch Hydrolyse oder Hydrogenolyse spaltet,
   und/oder eine Base oder Säure der Formel I in eines ihrer Salze umwandelt.

Gegenstand der Erfindung sind auch die Stereoisomeren (E, Z-Isomeren) sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Unter pharmazeutisch verwendbaren Derivaten versteht man z.B. die Salze der erfindungsgemäßen Verbindungen

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.

Darüberhinaus bedeutet der Ausdruck "therapeutisch wirksame Menge" eine Menge, die, verglichen zu einem entsprechenden Subjekt, das diese Menge nicht erhalten hat, folgendes zur Folge hat:
verbesserte Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder einer Störung.

Die Bezeichnung "therapeutisch wirksame Menge" umfaßt auch die Mengen, die wirkungsvoll sind, die normale physiologische Funktion zu erhöhen.

Gegenstand der Erfindung sind auch Mischungen der erfindungsgemäßen Verbindungen der Formel. I, z.B. Gemische zweier Diastereomerer oder Enantiomerer z.B. im Verhältnis 1:1, 1:2, 1:3, 1:4, 1:5, 1:10, 1:100 oder 1:1000.

Besonders bevorzugt handelt es sich dabei um Mischungen stereoisomerer Verbindungen, insbesondere liegen die erfindungsgemäßen Verbindungen als Racemat vor.

Für alle Reste, die mehrfach auftreten, gilt, daß deren Bedeutungen unabhängig voneinander sind.

Vor- und nachstehend haben die Reste bzw. Parameter R¹, R², R³, R⁴, R⁵, R⁶, R⁷, R⁸, R⁹, R¹⁰ und R¹¹ die bei der Formel I angegebenen Bedeutungen, falls nicht ausdrücklich etwas anderes angegeben ist.

A bedeutet Alkyl, ist unverzweigt (linear) oder verzweigt, und hat 1, 2, 3, 4, 5 oder 6 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2-oder 1,2,2-Trimethylpropyl, weiter bevorzugt z.B. Trifluormethyl.

A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.

Ac bedeutet Acetyl, Bn bedeutet Benzyl, Ms bedeutet -SO₂CH₃.
R¹ bedeutet vorzugsweise H, CHO oder Acetyl, besonders bevorzugt H.
R² bedeutet vorzugsweise H.
R³, R⁴, R⁵, R⁶, R⁷, R⁸, R⁹, R¹⁰, R¹¹ bedeuten vorzugsweise, jeweils unabhängig voneinander H, A, Hal, OR¹² oder O-[C(R¹²)₂]ₙAr.
R³ bedeutet besonders bevorzugt H, A oder Hal.
R⁶ bedeutet besonders bevorzugt OH.
R⁸ bedeutet besonders bevorzugt OH, A, Phenoxy oder Hal.
R⁴, R⁵, R⁷, R⁹, R¹⁰, R¹¹ bedeuten besonders bevorzugt H oder A.
R⁷ R¹⁰, R¹¹ bedeuten auch besonders bevorzugt, jeweils unabhängig voneinander, H oder Hal.
R¹² bedeutet besonders bevorzugt H.

Ar bedeutet z.B. Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Hydroxyphenyl, o-, m- oder p-Nitrophenyl, o-, m- oder p-Aminophenyl, o-, m- oder p-(N-Methylamino)-phenyl, o-, m- oder p-(N-Methylaminocarbonyl)-phenyl, o-, m- oder p-Acetamidophenyl, o-, m- oder p-Methoxyphenyl, o-, m- oder p-Ethoxyphenyl, o-, m- oder p-Ethoxycarbonylphenyl, o-, m- oder p-(N,N-Dimethylamino)-phenyl, o-, m- oder p-(N,N-Dimethylaminocarbonyl)-phenyl, o-, m- oder p-(N-Ethylamino)-phenyl, o-, m- oder p-(N,N-Diethylamino)-phenyl, o-, m- oder p-Fluorphenyl, o-, m- oder p-Bromphenyl, o-, m- oder p- Chlorphenyl, o-, m- oder p-(Methyl-sulfonamido)-phenyl, o-, m- oder p-(Methylsulfonyl)-phenyl, o-, m- oder p-Cyanphenyl, o-, m- oder p-Ureidophenyl, o-, m- oder p-Formylphenyl, o-, m- oder p-Acetylphenyl, o-, m- oder p-Aminosulfonylphenyl, o-, m- oder p-Carboxyphenyl, o-, m- oder p-Carboxymethyl-phenyl, o-, m- oder p-Carboxymethoxy-phenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,4- oder 2,5-Dinitrophenyl, 2,5- oder 3,4-Dimethoxyphenyl, 3-Nitro-4-chlorphenyl, 3-Amino-4-chlor-, 2-Amino-3-chlor-, 2-Amino-4-chlor-, 2-Amino-5-chlor- oder 2-Amino-6-chlorphenyl, 2-Nitro-4-N,N-dimethylamino- oder 3-Nitro-4-N,N-dimethylaminophenyl, 2,3-Diaminophenyl, 2,3,4-, 2,3,5-, 2,3,6-, 2,4,6- oder 3,4,5-Trichlorphenyl, 2,4,6-Trimethoxyphenyl, 2-Hydroxy-3,5-dichlorphenyl, p-lodphenyl, 3,6-Dichlor-4-aminophenyl, 4-Fluor-3-chlorphenyl, 2-fluor-4-bromphenyl, 2,5-Difluor-4-bromphenyl, 3-Brom-6-methoxyphenyl, 3-Chlor-6-methoxyphenyl, 3-Chlor-4-acetamidophenyl, 3-Fluor-4-methoxyphenyl, 3-Amino-6-methylphenyl, 3-Chlor-4-acetamidophenyl oder 2,5-Dimethyl-4-chlorphenyl.

Ar bedeutet vorzugsweise z.B. unsubstituiertes oder ein-, zwei- oder dreifach durch Hal, A, OR¹⁰, SO₂A, COOR¹⁰ oder CN substituiertes Phenyl, ganz besonders bevorzugt unsubstituiertes oder ein-, zwei- oder dreifach durch Hal und/oder A substituiertes Phenyl, insbesondere bedeutet Ar Phenyl.

Het bedeutet, ungeachtetet weiterer Substitutionen, z.B. 2- oder 3-Furyl, 2-oder 3-Thienyl, 1-, 2- oder 3-Pyrrolyl, 1-, 2, 4- oder 5-Imidazolyl, 1-, 3-, 4-oder 5-Pyrazolyl, 2-, 4- oder 5-Oxazolyl, 3-, 4- oder 5-Isoxazolyl, 2-, 4- oder 5-Thiazotyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder-5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, 1-, 2-, 4- oder 5-Benzimidazolyl, 1-, 2-, 3-, 4-, 5-, 6- oder 7-Indazolyl, 1-, 3-, 4-, 5-, 6- oder 7-Benzopyrazolyl, 2-, 4-, 5-, 6- oder 7-Benzoxazolyl, 3-, 4-, 5-, 6- oder 7- Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7-oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo-[1,4]oxazinyl, weiter bevorzugt 1,3-Benzodioxol-5-yl, 1,4-Benzodioxan-6-yl, 2,1,3-Berizothiadiazol-4- oder -5-yl oder 2,1,3-Benzoxadiazol-5-yl.

Die heterocyclischen Reste können auch teilweise oder vollständig hydriert sein.

Het kann also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3-oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3-oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-,-2-,-3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydro-benzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydrobenzofuranyl oder 2,3-Dihydro-2-oxo-furanyl.

Het bedeutet vorzugsweise einen einkernigen gesättigten, ungesättigten oder aromatischen Heterocyclus mit 1 bis 2 N- und/oder O-Atomen, der unsubstituiert oder ein-, zwei- oder dreifach durch A, Hal, OH und/oder OA substituiert sein kann.

Het bedeutet besonders bevorzugt einen einkernigen gesättigten Heterocyclus mit 1 bis 2 N- und/oder O-Atomen, der unsubstituiert oder ein- oder zweifach durch A substituiert sein kann.

In einer weiteren Ausführungsform bedeutet Het ganz besonders bevorzugt Pyrrolidinyl, Piperidinyl, Morpholinyl oder Piperazinyl.

In einer weiteren Ausführungsform bedeutet Het besonders bevorzugt unsubstituiertes oder ein-, zwei- oder dreifach durch A, Hal, OH und/oder OA substituiertes Furyl, Thienyl, Pyrrolyl, Imidazolyl, Pyridyl, Pyrimidinyl, Pyrazolyl, Thiazolyl, Indolyl, Pyrrolidinyl, Piperidinyl, Morpholinyl oder Piperazinyl.

Die Verbindungen der Formel I können ein oder mehrere chirale Zentren besitzen und daher in verschiedenen stereoisomeren Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Io ausgedrückt werden, die der Formel I entsprechen und worin die nicht näher bezeichneten Reste die bei der Formel I angegebene Bedeutung haben, worin jedoch
in Ia R¹ H oder CHO,
   R² H bedeuten;
in Ib R³, R⁴, R⁵, R⁶, R⁷,
   R⁸, R⁹, R¹⁰, R¹¹ jeweils unabhängig voneinander
   H, A, Hal, OR¹² oder O-[C(R¹²)₂]ₙAr
   bedeuten;
in Ic R⁶ OH bedeutet;
in Id R³ H, A oder Hal bedeutet;
in le R⁸ OH, A, Phenoxy oder Hal
   bedeutet;
in If R⁴, R⁵, R⁷, R⁹, R¹⁰, R¹¹ H oder A
   bedeuten;
in Ig R⁷, R¹⁰, R¹¹ jeweils unabhängig voneinander H oder Hal
   bedeuten;
in Ih Ar unsubstituiertes oder ein-, zwei- oder dreifach durch Hal und/oder A substituiertes Phenyl
   bedeutet;
in Ii Ar Phenyl bedeutet;
in Ij Het einen einkernigen gesättigten, ungesättigten oder aromatischen Heterocyclus mit 1 bis 2 N- und/oder O-Atomen, der unsubstituiert oder ein-, zwei- oder dreifach durch A, Hal, OH und/oder OA substituiert sein kann
   bedeutet;
in Ik Het einen einkernigen gesättigten Heterocyclus mit 1 bis 2 N- und/oder O-Atomen, der unsubstituiert oder ein- oder zweifach durch A substituiert sein kann,
   bedeutet;
in II Het unsubstituiertes oder ein-, zwei- oder dreifach durch A, Hal, OH und/oder OA substituiertes Furyl, Thienyl, Pyrrolyl, Imidazolyl, Pyridyl, Pyrimidinyl, Pyrazolyl, Thiazolyl, Indolyl, Pyrrolidinyl, Piperidinyl, Morpholinyl oder Piperazinyl,
   bedeutet;
in Im R¹ H oder CHO,
   R² H,
   R³, R⁴, R⁵, R⁶, R⁷,
   R⁸, R⁹, R¹¹, R¹¹ jeweils unabhängig voneinander
   H, A, Hal, OR¹² oder O-[C(R¹²)₂]ₙAr, bedeuten;
in In R¹ H oder CHO,
   R²H,
   R³ H, A oder Hal,
   R⁴, R⁵, R⁷, R⁹, R¹⁰, R¹¹ H oder A,
   R⁶ OH,
   R⁸ OH, A, Phenoxy oder Hal
   bedeuten;
in Io R¹ H, CHO oder Acetyl,
   R² H
   R³ H, A oder Hal,
   R⁴ R⁵, R⁷,
   R¹⁰, R¹¹ jeweils unabhängig voneinander H, A oder Hal,
   R⁶ OH,
   R⁸ OH, A, Phenoxy oder Hal,
   R⁹ H, Hal oder OA,
   R⁸ und R⁹ zusammen auch Methylendioxy, bedeuten;
sowie ihre pharmazeutisch verwendbaren Solvate, Salze und Stereoisomere, einschließlich deren Mischungen in allen Verhältnissen.

Besonders bevorzugt sind die Verbindungen der Formel I ausgewählt aus der Gruppe

| Nr. | Strukturformel |
|---|---|
| "A15" | 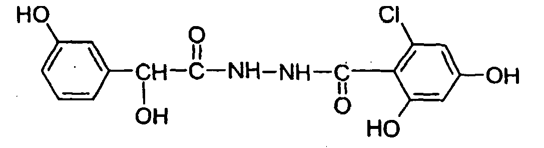 |
| | 2-Chlor-4,6-dihydroxy-benzoesäure-N'-[2-hydroxy-2-(3-hydroxy-phenyl)-acetyl]-hydrazid |
| "A16" | 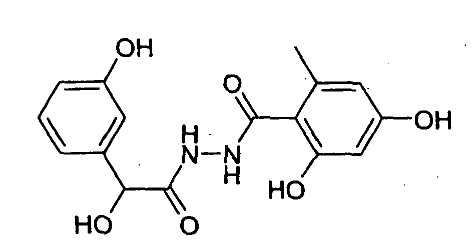 |
| | 2-Methyl-4,6-dihydroxy-benzoesäure-*N*'-[2-hydroxy-2-(3-hydroxy-phenyl)-acetyl]-hydrazid |
| "A17" | 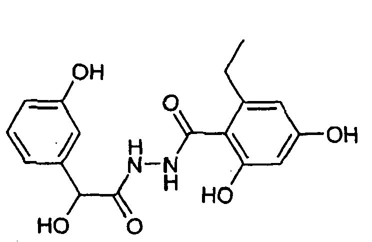 |
| | 2-Ethyl-4,6-dihydroxy-benzoesäure-*N*'-[2-hydroxy-2-(3-hydroxy-phenyl)-acetyl]-hydrazid |
| | |

Die erfindungsgemäßen Verbindungen und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Veriag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den erfindungsgemäßen Verbindungen umsetzt.

Die Ausgangsverbindungen sind in der Regel bekannt. Sind sie neu, so können sie aber nach an sich bekannten Methoden hergestellt werden.

Verbindungen der Formel I können vorzugsweise erhalten werden, indem man ein Hydrazid der Formel II mit einer Verbindung der Formel III umsetzt.

Die Umsetzung erfolgt nach Methoden, die dem Fachmann bekannt sind. Die Umsetzung erfolgt in der Regel in einem inerten Lösungsmittel, gegebenenfalls in Gegenwart eines säurebindenden Mittels vorzugsweise einer organischen Base wie DIPEA, Triethylamin, Dimethylanilin, Pyridin oder Chinolin oder eines Überschusses der Carboxykomponente der Formel III.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chlorform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Als Lösungsmittel besonders bevorzugt sind Wasser oder DMF.

Auch der Zusatz eines Alkali- oder Erdalkalimetall-hydroxids, -carbönats oder -bicarbonats oder eines anderen Salzes einer schwachen Säure der Alkali- oder Erdalkalimetalle, vorzugsweise des Kaliums, Natriums, Calciums oder Cäsiums kann günstig sein.

Die Reaktionszeit liegt je nach den angewendeten Bedingungen zwischen einigen Minuten und 14 Tagen, die Reaktionstemperatur zwischen etwa -30° und 140°, normalerweise zwischen -10° und 90°, insbesondere zwischen etwa 0° und etwa 70°.

In den Verbindungen der Formel III bedeutet L vorzugsweise Cl, Br, I oder eine freie oder eine reaktionsfähig abgewandelte OH-Gruppe wie z.B. ein aktivierter Ester, ein Imidazolid oder Alkylsulfonyloxy mit 1-6 C-Atomen (bevorzugt Methylsulfonyloxy oder Trifluormethylsulfonyloxy) oder Arylsulfonyloxy mit 6-10 C-Atomen (bevorzugt Phenyl- oder p-Tolylsulfonyloxy).

Derartige Reste zur Aktivierung der Carboxygruppe in typischen Acylierungsreaktionen sind in der Literatur (z.B. in den Standardwerken wie Houben-Weyl, Methoden der organischen Chemie, Georg-Thieme-Verlag, Stuttgart;) beschrieben.

Aktivierte Ester werden zweckmäßig in situ gebildet, z. B. durch Zusatz von HOBt oder N-Hydroxysuccinimid.

Verbindungen der Formel I können weiterhin vorzugsweise erhalten werden, indem man ein Hydrazid der Formel IV mit einer Verbindung der Formel V umsetzt.

Die Umsetzung erfolgt in der Regel in einem inerten Lösungsmittel, in Gegenwart eines säurebindenden Mittels vorzugsweise einer organischen Base wie DIPEA, Triethylamin, Dimethylanilin, Pyridin oder Chinolin oder eines Überschusses der Carboxykomponente der Formel V.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Düsopropylether, Tetrahydrofuran (THF) oder Dioxan; Glykolether wie Ethylenglykol-monomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitrover-bindungen wie Nitromethan oder Nitrobenzol; Ester wie Ethylacetat oder Gemische der genannten Lösungsmittel.

Auch der Zusatz eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein.

Die Reaktionszeit liegt je nach den angewendeten Bedingungen zwischen einigen Minuten und 14 Tagen, die Reaktionstemperatur zwischen etwa -30° und 140°, normalerweise zwischen -10° und 90°, insbesondere zwischen etwa 0° und etwa 70°.

In den Verbindungen der Formel V bedeutet L vorzugsweise Cl, Br, I oder eine freie oder eine reaktionsfähig abgewandelte OH-Gruppe wie z.B. ein aktivierter Ester, ein Imidazolid oder Alkylsulfonyloxy mit 1-6 C-Atomen (bevorzugt Methylsulfonyloxy oder Trifluormethylsulfonyloxy) oder Arylsulfonyloxy mit 6-10 C-Atomen (bevorzugt Phenyl- oder p-Tolylsulfonyloxy).

Derartige Reste zur Aktivierung der Carboxygruppe in typischen Acylierungsreaktionen sind in der Literatur (z.B. in den Standardwerken wie Houben-Weyl, Methoden der organischen Chemie, Georg-Thieme-Verlag, Stuttgart;) beschrieben.

Aktivierte Ester werden zweckmäßig in situ gebildet, z. B. durch Zusatz von HOBt oder N-Hydroxysuccinimid.

Verbindungen der Formel I können ferner erhalten werden, indem man einen Rest R¹, R², R³, R⁴, R⁵, R⁶, R⁷, R⁸, R⁹, R¹⁰ und/oder R¹¹ in einen anderen Rest R¹, R², R³, R⁴, R⁵, R⁶, R⁷, R⁸, R⁹, R¹⁰ und/oder R¹¹ umwandelt,
indem man z.B. einen Ether durch Hydrolyse oder Hydrogenolyse spaltet.

Die Spaltung eines Ethers erfolgt unter Methoden, wie sie dem Fachmann bekannt sind.

Eine Standardmethode zur Etherspaltung, z.B. eines Methylethers, ist die Verwendung von Bortribromid.

Hydrogenolytisch entfernbare Gruppen, z.B. die Spaltung eines Benzylethers, können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt.

Ester können z.B. mit Essigsäure oder mit NaOH oder KOH in Wasser, Wasser-THF oder Wasser-Dioxan bei Temperaturen zwischen 0 und 100° verseift werden.

### Pharmazeutische Salze und andere Formen

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formel I werden größtenteils konventionell hergestellt. Sofern die Verbindung der Formel I eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen der Formel I zählen ebenfalls dazu. Bei bestimmten Verbindungen der Formel I lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chforid, Chforbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, Iodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der Verbindungen der Formel I, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer Ionenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie (C₁-C₄) Alkylhalogeniden, z.B. Methyl-, Ethyl-, Isopropyl- und tert.-Butylchlorid, -bromid und -iodid; Di(C₁-C₄)Alkylsulfaten, z.B. Dimethyl-, Diethyl- und Diamylsulfat; (C₁₀-C₁₈)Alkylhalögeniden, z.B. Decyl-, Dodecyl-, Lauryl-, Myristyl- und Stearylchlorid, -bromid und -iodid; sowie Aryl-(C₁-C₄)Alkylhalogeniden, z.B. Benzylchlorid und Phenethylbromid, quartemisieren. Mit solchen Salzen können sowohl wasser- als auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Die Säureadditionssalze basischer Verbindungen der Formel werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen der Formel I mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine Verbindung der Formel I in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Erfindungsgemäße Verbindungen der Formel I können aufgrund ihrer Molekülstruktur chiral sein und können dementsprechend in verschiedenen enantiomeren Formen auftreten. Sie können daher in racemischer oder in optisch aktiver Form vorliegen.

Da sich die pharmazeutische Wirksamkeit der Racemate bzw. der Stereoisomeren der erfindungsgemäßen Verbindungen unterscheiden kann, kann es wünschenswert sein; die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktiven Säuren, wie die R- und S-Formen von Weinsäure, Diacetylweinsäure, Dibenzoylweinsäure, Mandelsäure, Äpfelsäure, Milchsäure, geeignet N-geschützte Aminosäuren (z.B. N-Benzoylprolin oder N-Benzolsulfonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieselgel fixierte chiral derivatisierte Methacrylatpolymere). Als Laufmittel eignen sich hierfür wäßrige oder alkoholische Lösungsmittelgemische wie z.B. Hexan/Isopropanol/ Acetonitril z.B. im Verhältnis 82:15:3.

Gegenstand der Erfindung ist ferner die Verwendung der Verbindungen und/oder ihrer physiologisch unbedenklichen Salze zur Herstellung eines Arzneimittels (pharmazeutische Zubereitung), insbesondere auf nichtchemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner Arzneimittel, enthaltend mindestens eine erfindungsgemäße Verbindung und/oder ihre pharmazeutisch verwendbaren Solvate und Stereoisomere, einschließlich deren Mischungen in allen Verhältnissen, sowie gegebenenfalls Träger- und/oder Hilfsstoffe.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder ÖI-in-Wasser-Flüssigemulsionen oder Wasser-in-ÖI-Flüssigemulsionen dargereicht werden.

So läßt sich beispielsweise bei der oralen Verabreichung in Form einer Tablette oder Kapsel die Wirkstoffkomponente mit einem oralen, nicht-toxischen und pharmazeutisch unbedenklichen inerten Trägerstoff, wie z.B. Ethanol, Glycerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindung-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmier mitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten Trägerstoff kombiniert und dann ohne Durchführung der Granulierungs-oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nicht toxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die erfindungsgemäßen Verbindungen sowie Salze und Solvate davon lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die erfindungsgemäßen Verbindungen sowie die Salze und Solvate können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Verneblem oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der vorliegenden Erfindung hängt von einer Reihe von Faktoren ab, einschließlich z.B. dem Alter und Gewicht des Menschen oder Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer erfindungsgemäßen Verbindung für die Behandlung im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes oder Solvats davon kann als Anteil der wirksamen Menge der erfindungsgemäßen Verbindung per se bestimmt werden. Es läßt sich annehmen, daß ähnliche Dosierungen für die Behandlung der anderen, obenerwähnten Krankheitszustände geeignet sind.

Gegenstand der Erfindung sind ferner Arzneimittel enthaltend mindestens eine erfindungsgemäße Verbindung und/oder ihre pharmazeutisch verwendbaren salze Solvate und Stereoisomere, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von
(a) einer wirksamen Menge an einer erfindungsgemäßen Verbindung und/oder ihrer pharmazeutisch verwendbaren salze Solvate und Stereoisomere, einschließlich deren Mischungen in allen Verhältnissen,
   und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer erfindungsgemäßen Verbindung und/oder ihrer pharmazeutisch verwendbaren salze Solvate und Stereoisomere, einschließlich deren Mischungen in allen Verhältnissen,
und einer wirksamen Menge eines weiteren Arzneimittelwirkstoffs gelöst oder in lyophylisierter Form vorliegt.

### VERWENDUNG

Die vorliegenden Verbindungen eignen sich als pharmazeutische Wirkstoffe für Säugetiere, insbesondere für den Menschen, bei der Behandlung von SGK-bedingten Krankheiten.

Gegenstand der Erfindung ist somit die Verwendung von Verbindungen nach Anspruch 1, sowie ihrer pharmazeutisch verwendbaren salze, Solvate und Stereoisomere, einschließlich deren Mischungen in allen Verhältnissen, zur Herstellung eines Arzneimittels zur Behandlung von Krankheiten, bei denen die Hemmung, Regulierung und/oder Modulation der Signaltransduktion von Kinasen eine Rolle spielt.
Bevorzugt ist hierbei SGK.

Bevorzugt ist die Verwendung von Verbindungen gemäß Anspruch 1, sowie ihrer pharmazeutisch verwendbaren salze, Solvate und Stereoisomere, einschließlich deren Mischungen in allen Verhältnissen, zur Herstellung eines Arzneimittels zur Behandlung von Krankheiten, die durch Inhibierung der SGK durch die Verbindungen nach Anspruch 1 beeinflußt werden.

Die vorliegende Erfindung umfasst die Verwendung der erfindungsgemäßen Verbindungen nach Anspruch 1 und/oder ihre physiologisch unbedenklichen Salze und Solvate zur Herstellung eines Arzneimittels zur Behandlung oder Vorbeugung von Diabetes (z.B. Diabetes mellitus, diabetische Nephropathie, diabetische Neuropathie, diabetische Angiopathie und Mikroangiopathie), Fettsucht, metabolisches Syndrom (Dyslipidämie), systemische und pulmonale Hypertonie, Herzkreislauferkrankungen (z.B. kardiale Fibrosen nach Myokardinfarkt, Herzhypertrophie und Herzinsuffizienz, Arteriosklerose) und Nierenerkrankungen (z.B. Glomerulosklerose, Nephrosklerose, Nephritis, Nephropathie, Störung der Elektrolytausscheidung), allgemein bei jeglicher Art von Fibrosen und entzündlichen Prozessen (z.B. Leberzirrhose, Lungenfibrose, fibrosierende Pankreatitis, Rheumatismus und Arthrosen, Morbus Crohn, chronische Bronchitis, Strahlenfibrose, Sklerodermitis, zystische Fibrose, Narbenbildung, Morbus Alzheimer).

Die erfindungsgemäßen Verbindungen können auch das Wachstum von Krebs, Tumorzellen und Tumormetastasen hemmen und sind deshalb für die Tumortherapie geeignet.

Die erfindungsgemäßen Verbindungen finden weiterhin Verwendung zur Behandlung von Koagulopathien, wie z.B. Dysfibrinogenämie, Hypoprokonvertinämie, Hämophilie B, Stuart-Prower-Defekt, Prothrombin-Komplex-Mangel, Verbrauchskoagulopathie, Hyperfibrinolyse, Immunokoagulopathie oder komplexer Koagulopathien, wie auch bei neuronaler Erregbarkeit, z.B. Epilepsie. Die erfindungsgemäßen Verbindungen können auch bei der Behandlung eines Glaukoms oder Katarakt therapeutisch eingesetzt werden.

Die erfindungsgemäßen Verbindungen finden ferner Verwendung bei der Behandlung bakterieller Infektionen sowie in einer antiinfektiösen Therapie. Die erfindungsgemäßen Verbindungen können auch zur Steigerung der Lernfähigkeit und Aufmerksamkeit therapeutisch eingesetzt werden.

Bevorzugt ist die Verwendung von Verbindungen gemäß Anspruch 1, sowie ihrer pharmazeutisch verwendbaren salze, Solvate und Stereoisomere, einschließlich deren Mischungen in allen Verhältnissen, zur Herstellung eines Arzneimittels zur Behandlung oder Vorbeugung von Diabetes, Fettsucht, metabolischem Syndrom (Dyslipidämie), systemischer und pulmonaler Hypertonie, Herzkreislauferkrankungen und Nierenerkrankungen, allgemein bei jeglicher Art von Fibrosen und entzündlichen Prozessen, Krebs, Tumorzellen, Tumormetastasen, Koagulopathien, neuronaler Erregbarkeit, Glaukom, Katarakt, bakteriellen Infektionen sowie in einer antünfektiösen Therapie, zur Steigerung der Lernfähigkeit und Aufmerksamkeit, sowie zur Behandlung und Prophylaxe von Zellalterung und Stress.

Bei Diabetes handelt es sich vorzugsweise um Diabetes mellitus, diabetische Nephropathie, diabetische Neuropathie, diabetische Angiopathie und Mikroangiopathie.

Bei Herzkreislauferkrankungen handelt es sich vorzugsweise um kardiale Fibrosen nach Myokardinfarkt, Herzhypertrophie, Herzinsuffizienz und Arteriosklerose.

Bei Nierenerkrankungen handelt es sich vorzugsweise um Glomerulosklerose, Nephrosklerose, Nephritis, Nephropathie und Störung der Elektrolytausscheidung.

Bei Fibrosen und entzündlichen Prozessen handelt es sich vorzugsweise um Leberzirrhose, Lungenfibrose, fibrosierende Pankreatitis, Rheumatismus und Arthrosen, Morbus Crohn, chronische Bronchitis, Strahlenfibrose, Sklerodermitis, zystische Fibrose, Narbenbildung, Morbus Alzheimer.

### ASSAYS

Die in den Beispielen beschriebenen erfindungsgemäßen Verbindungen wurden in den unten beschriebenen Assays geprüft, und es wurde gefunden, dass sie eine kinasehemmende Wirkung aufweisen. Weitere Assays sind aus der Literatur bekannt und könnten vom Fachmann leicht durchgeführt werden (siehe z.B. Dhanabal et al., Cancer Res. 59:189-197; Xin et al., J. Biol. Chem. 274:9116-9121; Sheu et al., Anticancer Res. 18:4435-4441; Ausprunk et al., Dev. Biol. 38:237-248; Gimbrone et al., J. Natl. Cancer Inst. 52:413-427; Nicosia et al., In Vitro 18:538- 549).

Die Hemmung der SGK1 Proteinkinase kann im Filterbindungsverfahren bestimmt werden.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.

| | |
|---|---|
| Massenspektrometrie (MS): | EI (Elektronenstoß-lonisation) M⁺ |
| | FAB (Fast Atom Bombardment) (M+H)⁺ |
| | ESI (Electrospray Ionization) (M+H)⁺ (wenn nichts anderes angegeben) |

### Beispiel 1

Die Herstellung von 2,4-Dihydroxy-6-methyl-benzoesäure-N'-[2-(3,4-difluor-phenyl)-2-hydroxy-acetyl]-hydrazid ("A1") erfolgt analog nachstehendem Schema 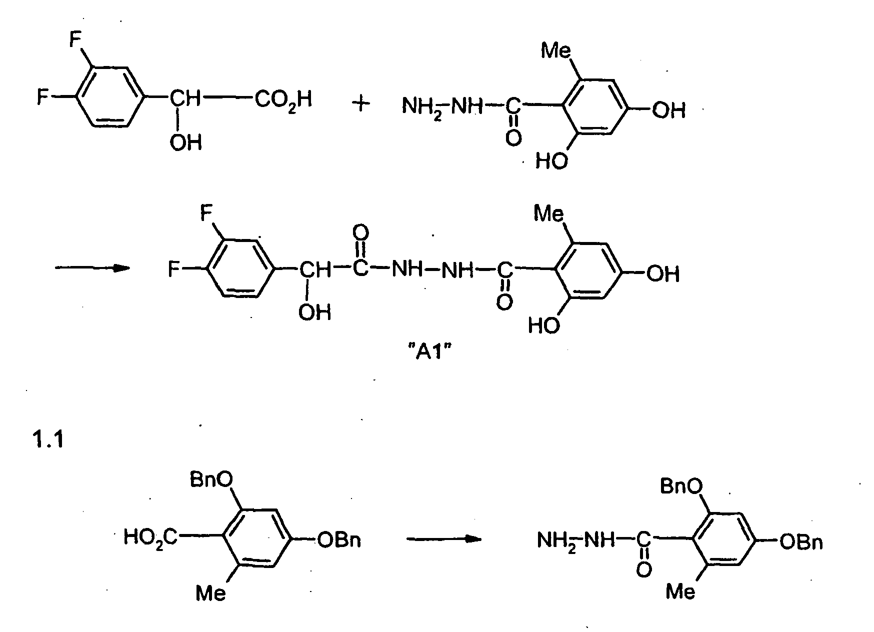 N₂H₅OH wird mit 2,4-Dibenzyloxy-6-methyl-benzoesäure monoacyliert. Ausbeute: 2,4-Dibenzyloxy-6-methyl-benzoesäure-hydrazid (63 %); F. 136-137°. 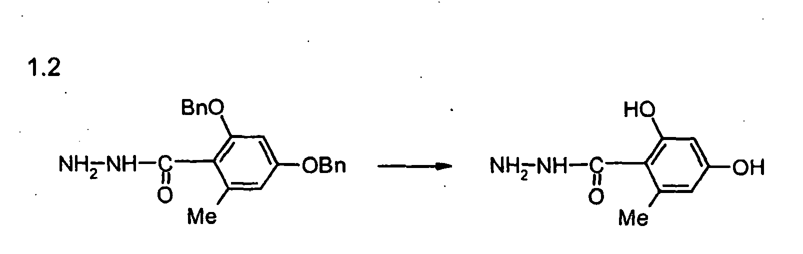 2,4-Dibenzyloxy-6-methyl-benzoesäure-hydrazid wird hydriert. Ausbeute: 2,4-Dihydroxy-6-methyl-benzoesäure-hydrazid (89 %); F. 226° (Zersetzung).
1.3 282 mg 3,4-Difluormandelsäure, 410 mg 2,4-Dihydroxy-6-methyl-benzoesäure-hydrazid, 431 mg 1-Ethyl-3-(3-dimethylaminopropyl)-carbodiimide (WSCD) und 164 mg 1-Hydroxybenzotriazole (HOBt) werden
3 h in 1.5 ml DMF gerührt. Man arbeitet wie üblich auf und chromatographiert über Kieselgel. Die einheitlichen Fraktionen werden vereinigt, eingeengt und aus EtOAc/Petrolether umkristallisiert. Ausbeute 250 mg "A1" (46%), F. 220 C.

Analog werden die nachstehenden Verbindungen erhalten

| Nr. | Strukturformel Name | F. [°C] |
|---|---|---|
| "A2" | 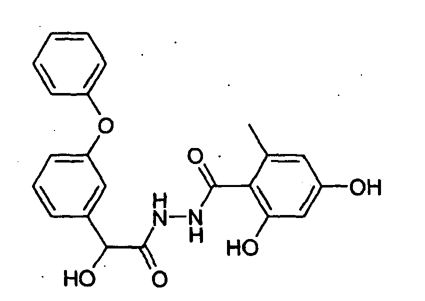 | 177-178 |
| | 2,4-Dihydroxy-6-methyl-benzoesäure-N'-[2-hydroxy-2-(3-phenoxy-phenyl)-acetyl]-hydrazid | |
| "A3" | 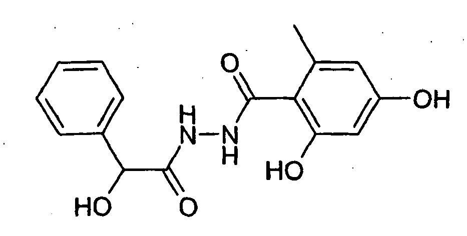 | 188-189 |
| | 2,4-Dihydroxy-6-methyl-benzoesäure-N'-(2-hydroxy-2-phenyl-acetyl)-hydrazid | |
| "A4" | 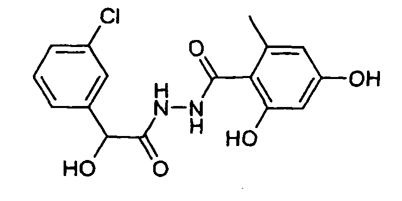 | 215 |
| | 2,4-Dihydroxy-6-methyl-benzoesäure-*N*'-[2-hydroxy-2-(3-chlor-phenyl)-acetyl]-hydrazid | |
| "A5" | 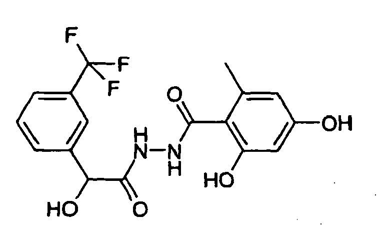 | 226 |
| | 2,4-Dihydroxy-6-methyl-benzoesäure-*N*'-[2-hydroxy-2-(3-trifluormethyl-phenyl)-acetyl]-hydrazid | |
| "A6" | 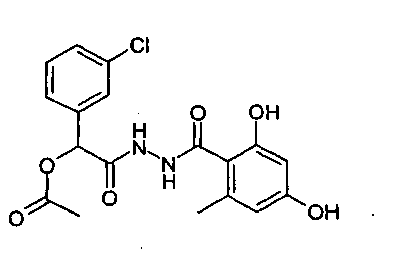 | 98-100 |
| | 2,4-Dihydroxy-6-methyl-benzoesäure-*N*'-[2-acetoxy-2-(3-chlor-phenyl)-acetyl]-hydrazid | |
| "A7" | 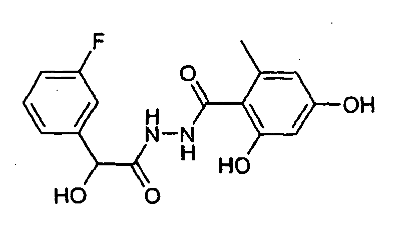 | 215-216 |
| | 2,4-Dihydroxy-6-methyl-benzoesäure-*N*'-[2-hydroxy-2-(3-fluor-phenyl)-acetyl]-hydrazid | |
| "A8" | 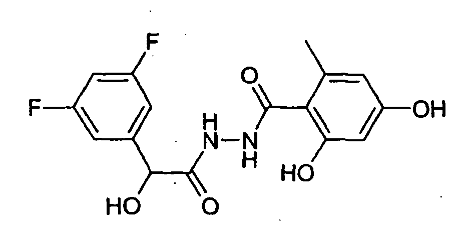 | 215-217 |
| | 2,4-Dihydroxy-6-methyl-benzoesäure-*N*'[2-hydroxy-2-(3,5-difluor-phenyl)-acetyl]-hydrazid | |
| "A9" | 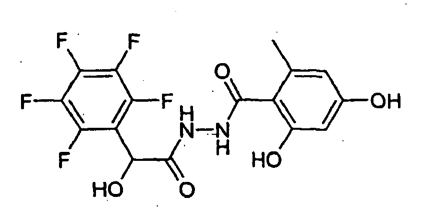 | 224-225 |
| | 2,4-Dihydroxy-6-methyl-benzoesäure-*N*'-[2-hydroxy-2-(2,3,4,5,6-pentafluor-phenyl)-acetyl]-hydrazid | |
| "A10" | 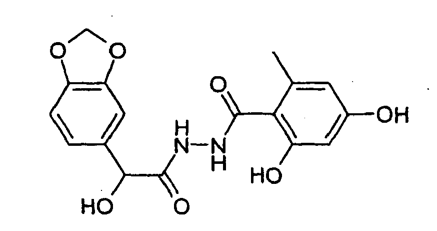 | 215-216 |
| | 2,4-Dihydroxy-6-methyl-benzoesäure-*N*'-(2-benzo[1,3]dioxol-5-yl-2-hydroxy-acetyl)-hydrazid | |
| "A11" | 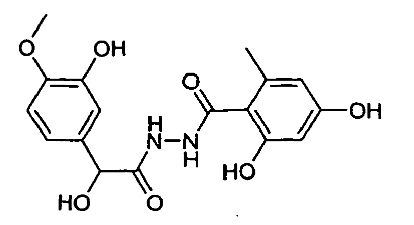 | 126-128 |
| | 2,4-Dihydroxy-6-methyl-benzoesäure-*N*'-[2-hydroxy-2-(3-hydroxy-4-methoxy-phenyl)-acetyl]-hydrazid | |
| "A12" | 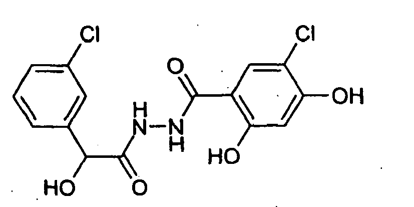 | 221-222 |
| | 2,4-Dihydroxy-5-chlor-benzoesäure-*N*'-[2-hydroxy-2-(3-chlor-phenyl)-acetyl]-hydrazid | |
| "A13" | 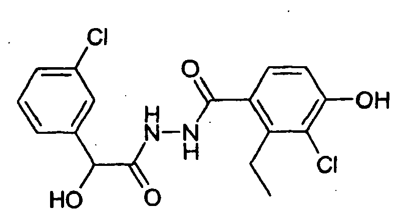 | 164-165 |
| | 3-Chlor-2-ethyl-4-hydroxy-benzoesäure-*N*'-[2-hydroxy-2-(3-chlor-phenyl)-acetyl]-hydrazid | |
| "A14" | 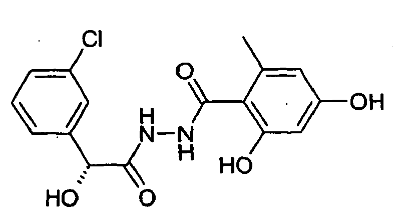 | 234-235 |
| | 2,4-Dihydroxy-6-methyl-benzoesäure-*N*'-[(R)-2-(3-chlor-phenyl)-2-hydroxy-acetyl]-hydrazid | |
| "A19" | 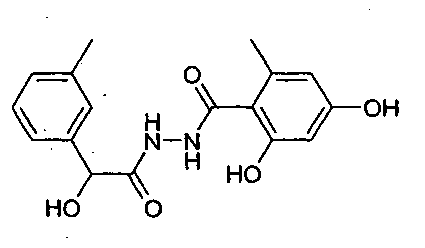 | 222-223 |
| | 2,4-Dihydroxy-6-methyl-benzoesäure-*N*'-[2-hydroxy-2-(3-methyl-phenyl)-acetyl]-hydrazid | |
| "A20" | 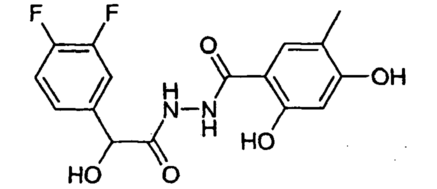 | 202-203 |
| | 2,4-Dihydroxy-5-methyl-benzoesäure-*N*'-[2-hydroxy-2-(3,4-difluor-phenyl)-acetyl]-hydrazid | |
| "A21" | 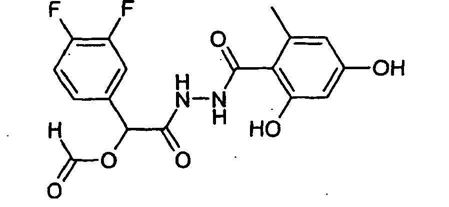 | 92-93 (Zersetzung) |
| | 2,4-Dihydroxy-6-methyl-benzoesäure-*N*'-[2-formyloxy-2-(3,4-difluor-phenyl)-acetyl]-hydrazid | |
| "A22" | 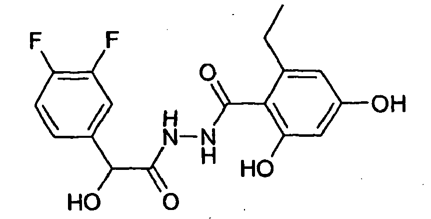 | 189-191 |
| | 2,4-Dihydroxy-6-ethyl-benzoesäure-*N*'-[2-hydroxy-2-(3,4-difluor-phenyl)-acetyl]-hydrazid | |
| "A23" | 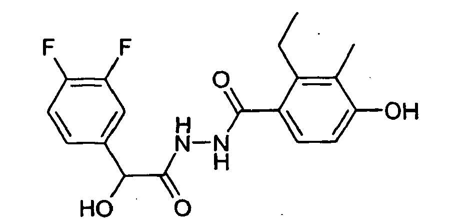 | 191 |
| | 4-Hydroxy-2-ethyl-3-methyl-benzoesäure-*N*'-[2-hydroxy-2-(3,4-difluor-phenyl)-acetyl]-hydrazid | |

### Beispiel 2

Herstellung von 2-Chlor-4,6-dihydroxy-benzoesäure-*N*'-[2-hydroxy-2-(3-hydroxy-phenyl)-acetyl]-hydrazid ("A15") 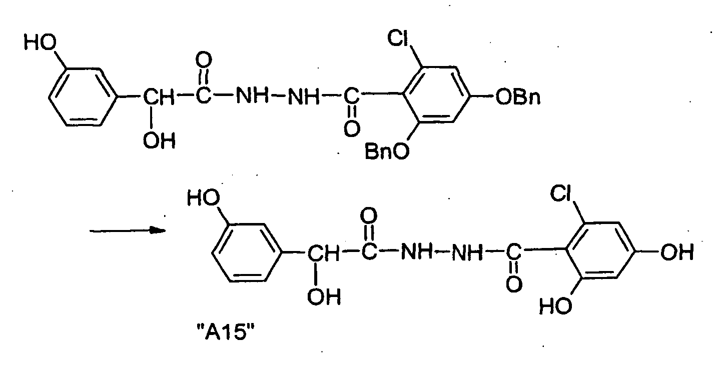 720 mg 2-Chlor-4,6-dibenzyloxy-benzoesäure-*N*'-[2-hydroxy-2-(3-hydroxyphenyl)-acetyl]-hydrazid in 20 ml THF + 0.2 ml 32%ige HCl werden mit 360 mg Pd/C mit der berechneten Menge H₂ hydriert. Die Hydrierlösung wird eingeengt und über Kieselgel chromatographiert. Die einheitlichen Fraktionen werden vereinigt, eingeengt und aus EtOAc umkristallisiert. Ausbeute 310 mg (65%) "A15", F. 230-231°.

Analog erhält man die nachstehenden Verbindungen

| Nr. | Strukturformel | F. [°C] |
|---|---|---|
| "A16" | 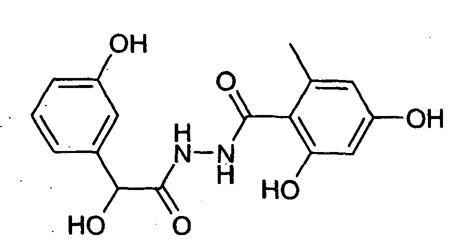 | 214-215 |
| | 2-Methyl-4,6-dihydroxy-benzoesäure-*N*'-[2-hydroxy-2-(3-hydroxy-phenyl)-acetyl]-hydrazid | |
| "A17" | 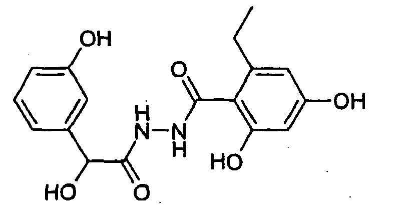 | 233-234 |
| | 2-Ethyl-4,6-dihydroxy-benzoesäure-*N*'-[2-hydroxy-2-(3-hydroxy-phenyl)-acetyl]-hydrazid | |
| | | |

### Beispiel 3

Herstellung von 2-Methyl-4,6-dihydroxy-benzoesäure-*N*'-(2-hydroxy-2-phenyl-acetyl)-hydrazid ("A18") 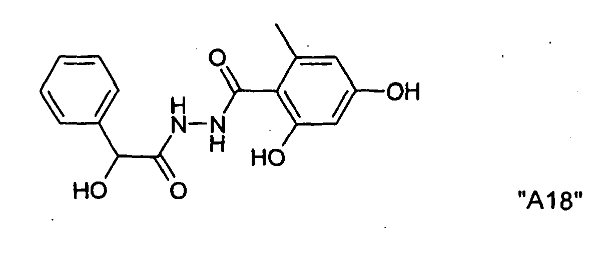 1,6 g 2,4-Dihydroxy-6-methyl-benzoesäure werden mit 4 ml SOCl₂ refluxiert bis eine klare Lösung entsteht. Das SOCl₂ wird abgezogen, anschließend wird noch 2 x mit CH₂Cl₂ zur Trockne eingedampft. Das Säurechlorid wird nun in 3 ml DMF gelöst und 1,14 g Mandelsäurehydrazid zugefügt. Nach 2-stündigem Rühren bei Raumtemperatur wird auf H₂O gegeben, mit EtOAc extrahiert, getrocknet und auf ein kleines Volumen eingeengt. Ausbeute 1,49 g (50%) "A18", F. 188-189°.

### Alternative Herstellung (Beispiel 4):

1,82 g 2,4-Dihydroxy-6-methyl-benzoesäure-hydrazid werden in 10 ml DMF gelöst. Dazu gibt man langsam 1,71 g Mandelsäurechlorid. Nach 2-stündigem Rühren bei Raumtemperatur wird auf H₂O gegeben, mit EtOAc extrahiert, getrocknet und auf ein kleines Volumen eingeengt. Ausbeute 2,16 g (68%) "A18", F. 188-189°.

### Synthesen von Vorstufen

### Beispiel 5

### 2,4-Dibenzyloxy-6-methyl-benzoesäure-N'-[2-hydroxy-2-(3-hydroxyphenyl)-acetyl]-hydrazid

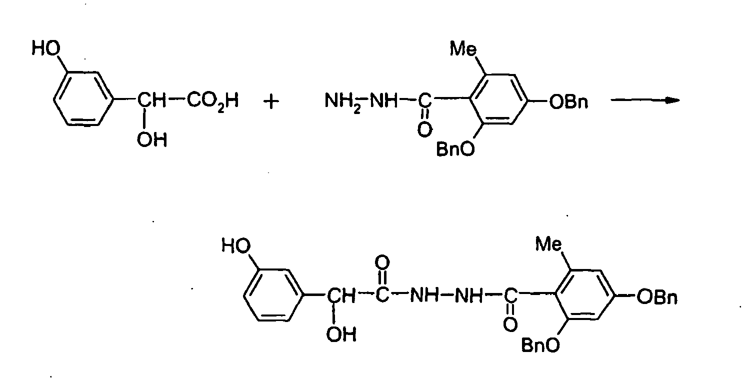

Die Substanz wird aus 2,4-Dibenzyloxy-6-methyl-benzoesäure-hydrazid und 3-Hydroxymandelsäure in 47%iger Ausbeute analog Beispiel 1 hergestellt, F. 181-182° (aus Me₂COH/ Et₂O).

Analog erhält man die Verbindungen

2-Chlor-4,6-dibenzyloxy-benzoesäure-*N'*-[2-hydroxy-2-(3-hydroxy-phenyl)-acetyl]-hydrazid, F. 160-162° (aus Me₂COH/ Et₂O), 61 % Ausbeute und 2,4-Dibenzyloxy-6-ethyl-benzoesäure-*N'*-[2-hydroxy-2-(3-hydroxy-phenyl)-acetyl]-hydrazid, F. 205-206° (aus Me₂COH/ Et₂O), 90 % Ausbeute.

### Beispiel 6

### 2,4-Bis-benzyloxy-6-ethyl-benzaldehyd

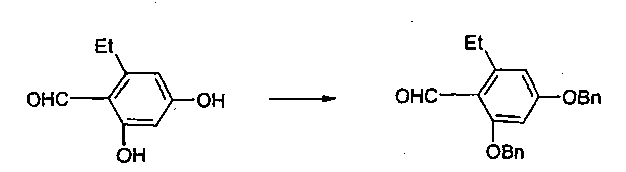

1,9 g Dihydroxy-6-ethyl-benzaldehyd, 3,48 ml Benzylchlorid und 4,7 g K₂CO₃ werden in 5,2 ml DMF 2h bei 90° gerührt. Der Ansatz wird mit EtOAc verdünnt und mit Wasser gewaschen. Die organische Phase wird mit Na₂SO₄ getrocknet, eingeengt und über Kieselgel chromatographiert. Die einheitlichen Fraktionen werden vereinigt und abgezogen. Der ölige Rückstand verfestigt sich beim Stehen nach einigen Tagen. Behandeln mit Ether/Petrolether 1:1 erbringen 3,5 g (88%) 2,4-Bis-benzyloxy-6-ethylbenzaldehyd, das sich an der Luft verfärbt.

3-Chlor-2-ethyl-4-hydroxy-benzoesäure wird analog benzyliert: 3-Chlor-2-ethyl-4-benzyloxy-benzoesäure wird in 78%iger Ausbeute erhalten, F. 208-210°.

### Beispiel 7

### 2,4-Bis-benzyloxy-6-ethyl-benzoesäure

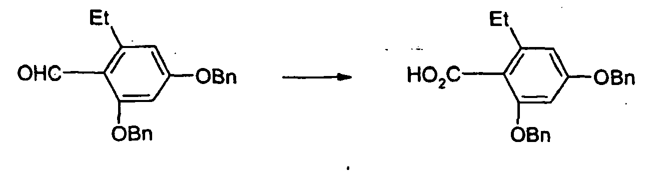

3,3 g 2,4-Bis-benzyloxy-6-ethyl-benzaldehyd werden in 63 ml DMSO gelöst. Dazu gibt man langsam unter Eiskühlung eine Lösung von 7,9 g NaClO₂ und 7,9 g NaHCO₃ in 32 ml Wasser, wobei die Temperatur 40°C nicht übersteigt. Es wird noch 2 h nachgerührt, bevor mit weiterem Wasser verdünnt und 2x mit EtOAc extrahiert wird. Die vereinigten organischen Phasen werden mit Wasser gewaschen, mit Na₂SO₄ getrocknet, eingeengt, über Kieselgel chromatographiert und aus Me₂COH kristallisiert:
Ausbeute 2,21 g (64%) 2,4-Bis-benzyloxy-6-ethyl-benzoesäure, F. 126-127°.

### Analog werden hergestellt:

2,4-Bis-benzyloxy-6-chlor-benzoesäure, F. 135-136° (35%) und 3-Chlor-2-ethyl-4-hydroxy-benzoesäure aus 3-Chlor-2-ethyl-4-hydroxybenzaldehyd: Ausbeute-51%, F. 138-139°.

### Beispiel 8

### 2,4-Dibenzyloxy-6-ethyl-benzoesäure-hydrazid

Die Substanz wird nach der in Beispiel 1 dargestellten Methode aus 2,4-Bis-benzyloxy-6-ethyl-benzoesäure und Hydraziniumhydroxid hergestellt: Ausbeute 75%, F. 140-141°.

Analog hergestellt wird auch 2,4-Dibenzyloxy-6-chlor-benzoesäure-hydrazid: Ausbeute 61%, F. 166-167° und
4-Benzyloxy-3-chlor-2-ethyl-benzoesäure-hydrazid. Ausbeute 85%, F. 182-184°.

### Beispiel 9

### 2,4-Bis-benzyloxy-6-chlor-benzaldehyd

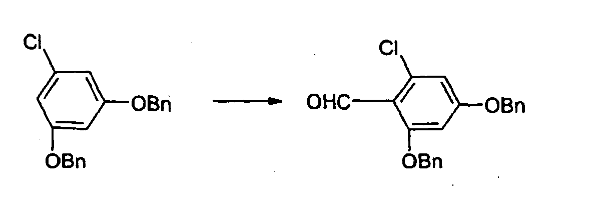

12 g Chlor-3,5-dibenzyloxy-benzol werden in 40 ml DMF gelöst. Bei 5-10° werden langsam 12 ml POCl₃ zugetropft. Man läßt 90 min bei Raumtemperatur, dann über Nacht bei 80 °C weiterreagieren. Die Lösung wird am Rotavapor eingeengt, dann auf 200 ml Eiswasser gegeben. Es wird 3x mit EtOAc extrahiert, mit Wasser gewaschen, getrocknet und eingeengt. Kieselgelchromatographie erbringt 8,2 g (63%) 2,4-Bis-benzyloxy-6-chlor-benzaldehyd, welches aus (Me₂C)₂O kristallisiert, F. 85-86°.

### Beispiel 10

### Acetoxy-(3-chlor-phenyl)-essigsäure

1 g racemische 3-Chlormandelsäure wird mit 2 ml Acetylchlorid vermischt. Es bildet sich eine klare Lösung, die nach 2 h am Rotavapor eingeengt und mit (Me₂C)₂O/Petrolether kristallisiert wird. Ausbeute 670 mg (55%), F. 118°.

### Beispiel 11

### 3-Chlor-2-ethyl-4-hydroxy-benzoesäure-hydrazid

Die Verbindung wird durch Hydrierung von 300 mg 3-Chlor-2-ethyl-4-benzyloxy-benzoesäure-hydrazid in 10 ml MeOH und 145 µl 32%ige HCl an Pd/Cu hergestellt. Ausbeute 165 mg (78%), 233-235° (EtOAc/MeCN).

Analog wird hergestellt: 5-Chlor-2,4-dihydroxy-benzoesäure-hydrazid, Ausbeute 84%, F. 260°.

### Beispiel 12

### 3-Chlor-2-ethyl-4-hydroxy-benzaldehyd

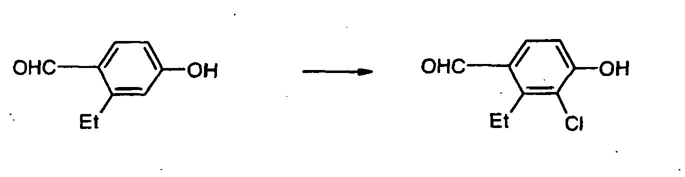

Zu 4 g 2-Ethyl-4-hydroxybenzaldehyd, gelöst in 80 ml CHCl₃ und 1,5 ml konz. HCl, werden 3,4 g *N*-Chlorsuccinimid in 40 ml CHCl₃ über einen Zeitraum 45 min zugetropft. Es wird 1 h nachgerührt, mit Wasser gewaschen, getrocknet und eingeengt.

Kieselgelchromatographie erbringt 1 g (20%) 3-Chlor-2-ethyl-4-hydroxybenzaldehyd (F. 85°, polare Substanz an Kieselgel bei Ether/Petrolether 1:1) neben 5-Chlor-2-ethyl-4-hydroxy-benzaldehyde (F. 83°, mittelpolar) und 3,5-Dichlor-2-ethyl-4-hydroxy-benzaldehyd (F. 117-118°, unpolar).

Die nachfolgenden Beispiele betreffen pharmazeutische Zubereitungen:

### Beispiel A: Injektionsgläser

Eine Lösung von 100 g eines erfindungsgemäßen Wirkstoffes und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines erfindungsgemäßen Wirkstoffes mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines erfindungsgemäßen Wirkstoffes, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines erfindungsgemäßen Wirkstoffes mit 99,5 g Vaseline unter aseptischen Bedingungen.

### Beispiel E: Tabletten

Ein Gemisch von 1 kg Wirkstoff, 4 kg Lactose, 1,2 kg Kartoffelstärke, 0,2 kg Talk und 0,1 kg Magnesiumstearat wird in üblicher Weise zu Tabletten verpreßt, derart, daß jede Tablette. 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff werden in üblicher Weise in Hartgelatinekapseln gefüllt, so daß jede Kapsel 20 mg des Wirkstoffs enthält.

### Beispiel H: Ampullen

Eine Lösung von 1 kg eines erfindungsgemäßen Wirkstoffes in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel I 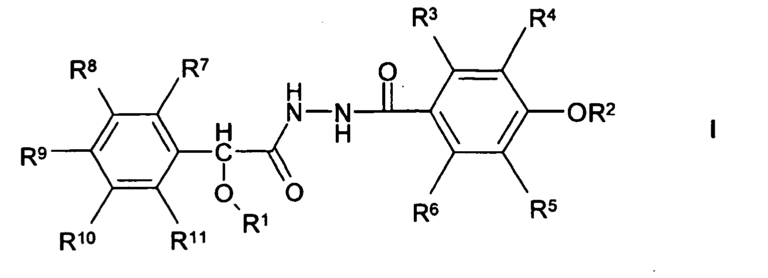 worin
R¹, R² jeweils unabhängig voneinander H, CHO oder Acetyl, R³, R⁴, R⁵, R⁶, R⁷,
R⁸, R⁹, R¹⁰, R¹¹ jeweils unabhängig voneinander
H, A, OSO₂A, HaI, NO₂, OR¹², N(R¹²)₂, CN, O-COA, -[C(R¹²)₂]ₙCOOR¹², O-[C(R¹²)₂]ₒCOOR¹², SO₃H, -[C(R¹²)₂]ₙAr, -CO-Ar, O-[C(R¹²)₂]ₙAr, -[C(R¹²)₂]ₙHet, -[C(R¹²)₂]ₙC=CH, O-[C(R¹²)₂]ₙC≡CH, -[C(R¹²)₂]ₙCON(R¹²)₂, -[C(R¹²)₂]ₙCONR¹²N(R¹²)₂, O-[C(R¹²)₂]ₙCON(R¹²)₂, O-[C(R¹²)₂]ₒCONR¹²N(R¹²)₂, NR¹²COA, NR¹²CON(R¹²)₂, NR¹²SO₂A, N(SO₂A)₂, COR¹², S(O)mAr, SO₂NR¹² oder S(O)ₘA,
R³ und R⁴ zusammen auch CH=CH-CH=CH,
R³ und R⁴, R⁷ und R⁸
oder R⁸ und R⁹ zusammen auch Alkylen mit 3, 4 oder 5 C-Atomen,
worin eine oder zwei CH₂-Gruppen durch Sauerstoff ersetzt sein können,
A unverzweigtes oder verzweigtes Alkyl mit 1-6 C-Atomen,
worin 1-7 H-Atome durch F ersetzt sein können,
oder cylisches Alkyl mit 3-7 C-Atomen,
Ar unsubstituiertes oder ein-, zwei- oder dreifach durch Hal, A,
OR¹², N(R¹²)₂, NO₂, CN, Phenyl, CON(R¹²)₂, NR¹²COA, NR¹²CON(R¹²)₂, NR¹²SO₂A, COR¹², SO₂N(R¹²)₂, S(O)ₘA, -[C(R¹² )₂]ₙ-COOR¹² und/oder -O[C(R¹²)₂]ₒ-COOR¹²
substituiertes Phenyl, Naphthyl oder Biphenyl,
Het einen ein- oder zweikernigen gesättigten, ungesättigten oder
aromatischen Heterocyclus mit 1 bis 4 N-, O- und/oder S-Atomen, der ein-, zwei- oder dreifach durch Hal, A, OR¹², N(R¹²)₂, NO₂, CN, COOR¹², CON(R¹²)₂, NR¹²COA, NR¹²SO₂A, COR¹², SO₂NR¹², S(O)ₘA, =S, =NR¹² und/oder =O (Carbonylsauerstoff) substituiert sein kann,
R¹² H oder A,
Hal F, Cl, Br oder l,
m 0, 1 oder 2,
n 0, 1, 2 oder 3,
o 1, 2 oder 3
bedeuten,
sowie ihre pharmazeutisch verwendbaren Salze, Solvate und Stereoisomere, einschließlich deren Mischungen in allen Verhältnissen.

2. Verbindungen nach Anspruch 1, worin
R¹ H oder CHO,
R² H bedeuten,
sowie ihre pharmazeutisch verwendbaren Salze, Solvate und Stereoisomere, einschließlich deren Mischungen in allen Verhältnissen.

3. Verbindungen nach Anspruch 1 oder 2, worin
R³, R⁴, R⁵, R⁶, R⁷,
R⁸, R⁹, R¹⁰, R¹¹ jeweils unabhängig voneinander
H, A, Hal, OR¹² oder O-[C(R¹²)₂]ₙAr
bedeuten,
sowie ihre pharmazeutisch verwendbaren Salze, Solvate und Stereoisomere, einschließlich deren Mischungen in allen Verhältnissen.

4. Verbindungen nach einem oder mehreren der Ansprüche 1-3, worin R⁶ OH bedeutet,
sowie ihre pharmazeutisch verwendbaren Salze, Solvate und Stereoisomere, einschließlich deren Mischungen in allen Verhältnissen.

5. Verbindungen nach einem oder mehreren der Ansprüche 1-4, worin R³ H, A oder Hal bedeutet,
sowie ihre pharmazeutisch verwendbaren Salze, Solvate und Stereoisomere, einschließlich deren Mischungen in allen Verhältnissen.

6. Verbindungen nach einem oder mehreren der Ansprüche 1-5, worin R⁸ OH, A, Phenoxy oder Hal bedeutet,
sowie ihre pharmazeutisch verwendbaren Salze, Solvate und Stereoisomere, einschließlich deren Mischungen in allen Verhältnissen.

7. Verbindungen nach einem oder mehreren der Ansprüche 1-6, worin R⁴, R⁵, R⁷, R⁹, R¹⁰, R¹¹ H oder A bedeuten,
sowie ihre pharmazeutisch verwendbaren Salze, Solvate und Stereoisomere, einschließlich deren Mischungen in allen Verhältnissen.

8. Verbindungen nach einem oder mehreren der Ansprüche 1-7, worin R⁷, R¹⁰, R¹¹ jeweils unabhängig voneinander H oder Hal bedeuten,
sowie ihre pharmazeutisch verwendbaren Salze, Solvate und Stereoisomere, einschließlich deren Mischungen in allen Verhältnissen.

9. Verbindungen nach einem oder mehreren der Ansprüche 1-8, worin Ar unsubstituiertes oder ein-, zwei- oder dreifach durch Hal
und/oder A substituiertes Phenyl bedeutet,
sowie ihre pharmazeutisch verwendbaren Salze, Solvate und Stereoisomere, einschließlich deren Mischungen in allen Verhältnissen.

10. Verbindungen nach einem oder mehreren der Ansprüche 1-9, worin Ar Phenyl bedeutet,
sowie ihre pharmazeutisch verwendbaren Salze, Solvate und Stereoisomere, einschließlich deren Mischungen in allen Verhältnissen.

11. Verbindungen nach einem oder mehreren der Ansprüche 1-10, worin Het einen einkernigen gesättigten, ungesättigten oder
aromatischen Heterocyclus mit 1 bis 2 N- und/oder O-Atomen, der unsubstituiert oder ein-, zwei- oder dreifach durch A, Hal, OH und/oder OA substituiert sein kann, bedeutet,
sowie ihre pharmazeutisch verwendbaren Salze, Solvate und Stereoisomere, einschließlich deren Mischungen in allen Verhältnissen.

12. Verbindungen nach einem oder mehreren der Ansprüche 1-11, worin Het einen einkernigen gesättigten Heterocyclus mit 1 bis 2 N- und/oder O-Atomen, der unsubstituiert oder ein- oder zweifach durch A substituiert sein kann,
bedeutet,
sowie ihre pharmazeutisch verwendbaren Salze, Solvate und Stereoisomere, einschließlich deren Mischungen in allen Verhältnissen.

13. Verbindungen nach einem oder mehreren der Ansprüche 1-12, worin Het unsubstituiertes oder ein-, zwei- oder dreifach durch A, Hal,
OH und/oder OA substituiertes Furyl, Thienyl, Pyrrolyl, Imidazolyl, Pyridyl, Pyrimidinyl, Pyrazolyl, Thiazolyl, Indolyl, Pyrrolidinyl, Piperidinyl, Morpholinyl oder Piperazinyl, bedeutet,
sowie ihre pharmazeutisch verwendbaren Salze, Solvate und Stereoisomere, einschließlich deren Mischungen in allen Verhältnissen.

14. Verbindungen nach einem oder mehreren der Ansprüche 1-13, worin R¹ H oder CHO,
R² H,
R³, R⁴, R⁵, R⁶, R⁷,
R⁸, R⁹, R¹⁰, R¹¹ jeweils unabhängig voneinander
H, A, Hal, OR¹² oder O-[C(R¹²)₂]ₙAr,
bedeuten,
sowie ihre pharmazeutisch verwendbaren Salze, Solvate und Stereoisomere, einschließlich deren Mischungen in allen Verhältnissen.

15. Verbindungen nach einem oder mehreren der Ansprüche 1-14, worin R¹ H oder CHO
R2 H,
R³ H, A oder Hal,
R⁴, R⁵, R⁷, R⁹, R¹⁰, R¹¹ H oder A,
R⁶ OH,
R⁸ OH, A, Phenoxy oder Hal
bedeuten,
sowie ihre pharmazeutisch verwendbaren Salze, Solvate und Stereoisomere, einschließlich deren Mischungen in allen Verhältnissen.

16. Verbindungen nach einem oder mehreren der Ansprüche 1-15, worin R¹ H oder CHO
R²H,
R³ H, A oder Hal,
R⁴, R⁵, R⁷, R⁹, R¹⁰, R¹¹ H, A oder Hal,
R⁸ und R⁹ zusammen auch Methylendioxy,
R⁶ OH,
R⁸ OH, A, Phenoxy oder Hal
bedeuten,
sowie ihre pharmazeutisch verwendbaren Salze, Solvate und Stereoisomere, einschließlich deren Mischungen in allen Verhältnissen.

17. Verbindungen nach einem oder mehreren der Ansprüche 1-16, worin
R¹ H, CHO oder Acetyl,
R² H
R³ H, A oder Hal,
R⁴, R⁵, R⁷,
R¹⁰, R¹¹ jeweils unabhängig voneinander H, A oder Hal,
R⁶ OH,
R⁸ OH, A, Phenoxy oder Hal,
R⁹ H, Hal oder OA,
R⁸ und R⁹ zusammen auch Methylendioxy,
bedeuten,
sowie ihre pharmazeutisch verwendbaren Salze, Solvate und Stereoisomere, einschließlich deren Mischungen in allen Verhältnissen.

18. Verbindungen nach Anspruch 1 ausgewählt aus der Gruppe
| Nr. | Strukturformel Name |
|---|---|
| "A1" | 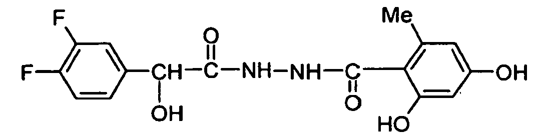 |
| | 2,4-Dihydroxy-6-methyl-benzoesäure-*N'*-[2-(3,4-difluor-phenyl)-2-hydroxy-acetyl]-hydrazid |
| "A2" | 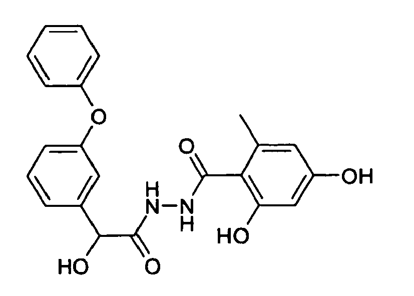 |
| | 2,4-Dihydroxy-6-methyl-benzoesäure-*N'*-[2-hydroxy-2-(3-phenoxy-phenyl)-acetyl]-hydrazid |
| "A3" | 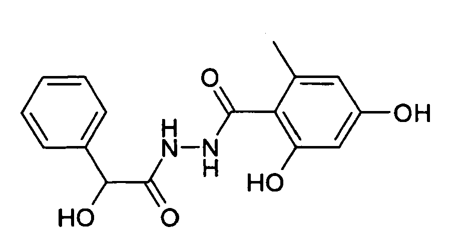 |
| | 2,4-Dihydroxy-6-methyl-benzoesäure-*N'*-(2-hydroxy-2-phenyl-acetyl)-hydrazid |
| "A4" | 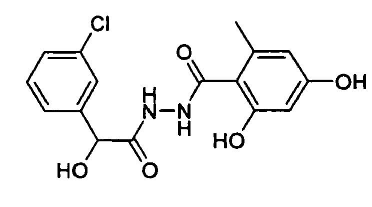 |
| | 2,4-Dihydroxy-6-methyl-benzoesäure-*N'*-[2-hydroxy-2-(3-chlor-phenyl)-acetyl]-hydrazid |
| "A5" | 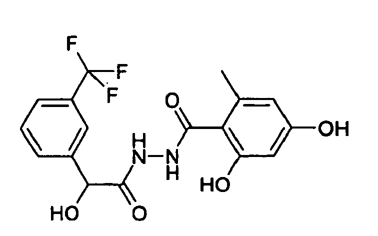 |
| | 2,4-Dihydroxy-6-methyl-benzoesäure-*N'*-[2-hydroxy-2-(3-trifl uormethyl-phenyl)-acetyl]-hyd razid |
| "A6" | 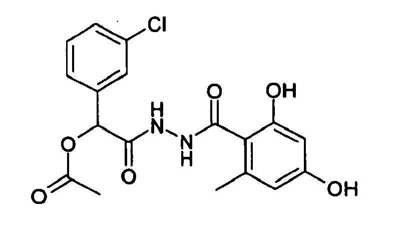 |
| | 2,4-Dihydroxy-6-methyl-benzoesäure-*N'*-[2-acetoxy-2-(3-chlor-phenyl)-acetyl]-hydrazid |
| | |
| "A7" | 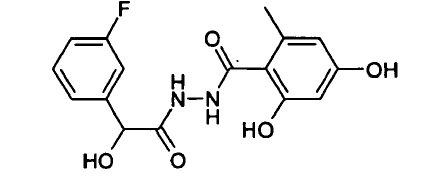 |
| | 2,4-Dihydroxy-6-methyl-benzoesäure-*N'*-[2-hydroxy-2-(3-fluor-phenyl)-acetyl]-hydrazid |
| "A8" | 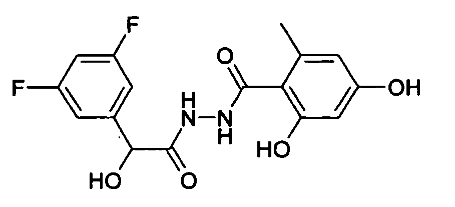 |
| | 2,4-Dihydroxy-6-methyl-benzoesäure-*N'*-[2-hydroxy-2-(3,5-difluor-phenyl)-acetyl]-hydrazid |
| "A9" | 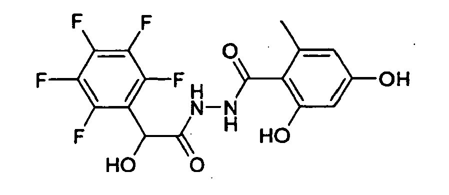 |
| | 2,4-Dihydroxy-6-methyl-benzoesäure-*N'*-[2-hydroxy-2-(2,3,4,5,6-pentafluor-phenyl)-acetyl]-hydrazid |
| "A10" | 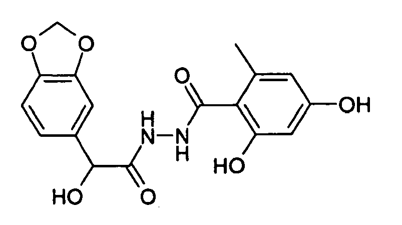 |
| | 2,4-Dihydroxy-6-methyl-benzoesäure-*N'*-(2-benzo[1,3]dioxol-5-yl-2-hydroxy-acetyl)-hydrazid |
| "A11" | 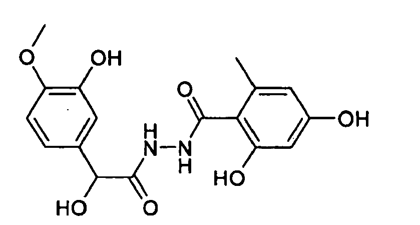 |
| | 2,4-Dihydroxy-6-methyl-benzoesäure-*N'*-[2-hydroxy-2-(3-hydroxy-4-methoxy-phenyl)-acetyl]-hydrazid |
| "A12" | 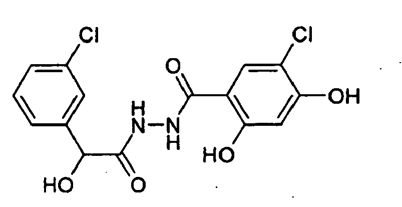 |
| | 2,4-Dihydroxy-5-chlor-benzoesäure-*N'*-[2-hydroxy-2-(3-chlor-phenyl)-acetyl]-hydrazid |
| "A13" | 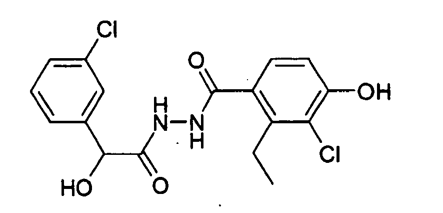 |
| | 3-Chlor-2-ethyl-4-hydroxy-benzoesäure-*N'*-[2-hydroxy-2-(3-chlor-phenyl)-acetyl]-hydrazid |
| "A14" | 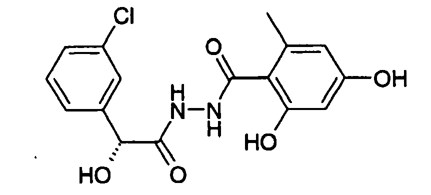 |
| | 2,4-Dihydroxy-6-methyl-benzoesäure-*N'*-[(R)-2-(3-chlor-phenyl)-2-hydroxy-acetyl]-hydrazid |
| "A15" | 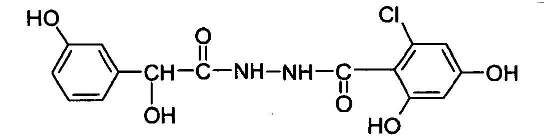 |
| | 2-Chlor-4,6-dihydroxy-benzoesäure-*N'*-[2-hydroxy-2-(3-hydroxy-phenyl)-acetyl]-hydrazid |
| "A16" | 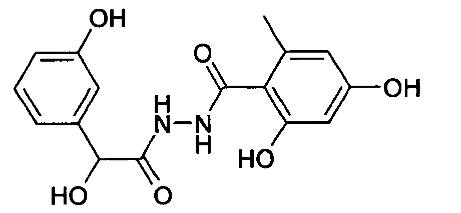 |
| | 2-Methyl-4,6-dihydroxy-benzoesäure-*N'*-[2-hydroxy-2-(3-hydroxy-phenyl)-acetyl]-hydrazid |
| "A17" | 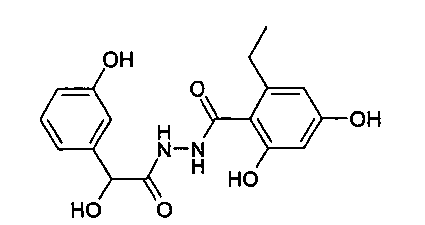 |
| | 2-Ethyl-4,6-dihydroxy-benzoesäure-*N'*-[2-hydroxy-2-(3-hydroxy-phenyl)-acetyl]-hydrazid |
| "A18" | 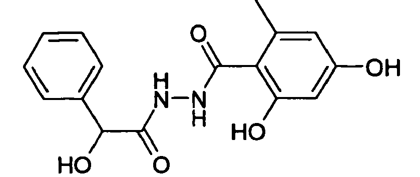 |
| | 2-Methyl-4,6-dihydroxy-benzoesäure-*N'*-(2-hydroxy-2-phenyl-acetyl)-hydrazid |
| "A19" | 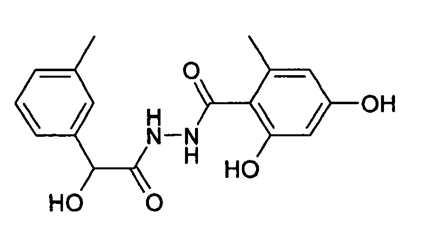 |
| | 2,4-Dihydroxy-6-methyl-benzoesäure-*N'*-[2-hydroxy-2-(3-methyl-phenyl)-acetyl]-hydrazid |
| "A20" | 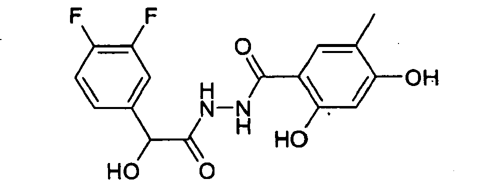 |
| | 2,4-Dihydroxy-5-methyl-benzoesäure-*N'*-[2-hydroxy-2-(3,4-difluor-phenyl)-acetyl]-hydrazid |
| "A21" | 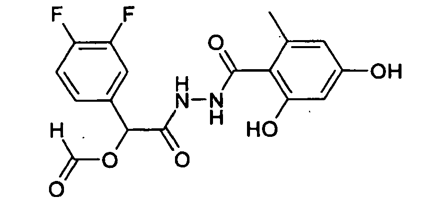 |
| | 2,4-Dihydroxy-6-methyl-benzoesäure-*N'*-[2-formyloxy-2-(3,4-difluor-phenyl)-acetyl]-hydrazid |
| "A22" | 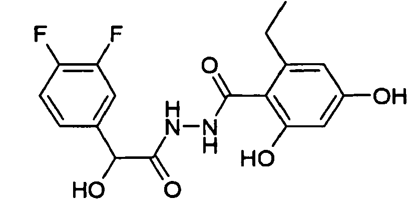 |
| | 2,4-Dihydroxy-6-ethyl-benzoesäure-*N'*-[2-hydroxy-2-(3,4-difluor-phenyl)-acetyl]-hydrazid |
| "A23" | 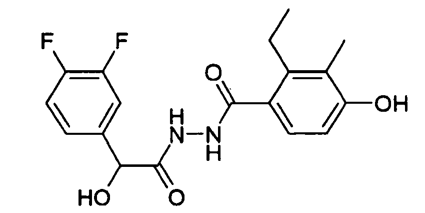 |
| | 4-Hydroxy-2-ethyl-3-methyl-benzoesäure-*N'*-[2-hydroxy-2-(3,4-difluor-phenyl)-acetyl]-hydrazid |
sowie ihre pharmazeutisch verwendbaren Salze, Solvate und Stereoisomere, einschließlich deren Mischungen in allen Verhältnissen.

19. Verfahren zur Herstellung von Verbindungen der Formel I nach den Ansprüchen 1-18 sowie ihrer pharmazeutisch verwendbaren Solvate, Salze und Stereoisomere, **dadurch gekennzeichnet, daß** man
a) eine Verbindung der Formel II 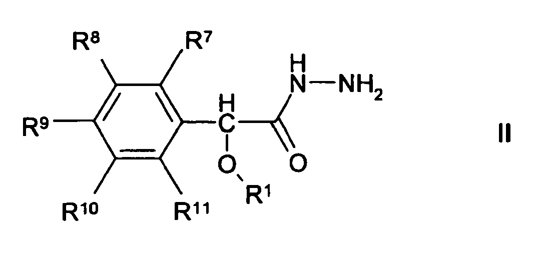 worin
R¹, R⁷, R⁸, R⁹, R¹⁰ und R¹¹ die in Anspruch 1 angegebenen Bedeutungen haben,
mit einer Verbindung der Formel III 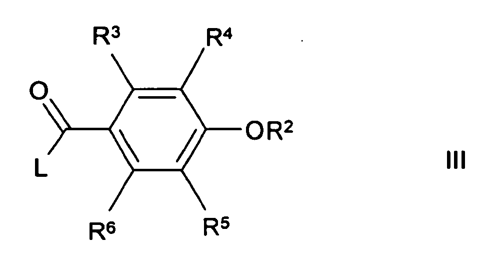 worin
L Cl, Br, I oder eine freie oder reaktionsfähig funktionell abgewandelte OH-Gruppe bedeutet und
R², R³, R⁴, R⁵ und R⁶ die in Anspruch 1 angegebenen Bedeutungen haben,
umsetzt,
oder
b) eine Verbindung der Formel IV 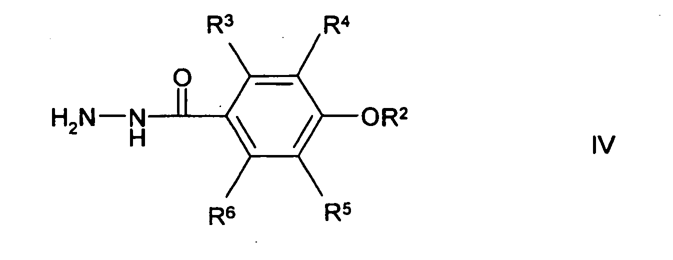 worin
R², R³, R⁴, R⁵ und R⁶ die in Anspruch 1 angegebenen Bedeutungen haben,
mit einer Verbindung der Formel V 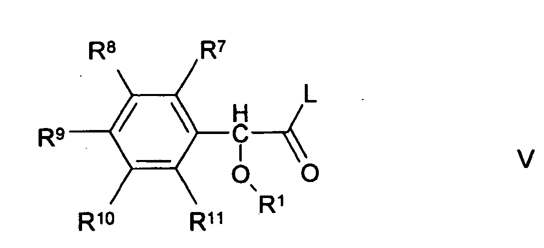 worin
L Cl, Br, I oder eine freie oder reaktionsfähig funktionell abgewandelte OH-Gruppe bedeutet und
R¹, R⁷, R⁸, R⁹, R¹⁰ und R¹¹ die in Anspruch 1 angegebenen Bedeutungen haben,
umsetzt,
oder
c) in einer Verbindung der Formel I einen Rest R¹, R², R³, R⁴, R⁵, R⁶, R⁷, R⁸, R⁹, R¹⁰ und/oder R¹¹ in einen anderen Rest R¹, R², R³, R⁴, R⁵, R⁶, R⁷, R⁸, R⁹, R¹⁰ und/oder R¹¹ umwandelt,
indem man einen Ether durch Hydrolyse oder Hydrogenolyse spaltet, und/oder eine Base oder Säure der Formel I in eines ihrer Salze umwandelt.

20. Arzneimittel, enthaltend mindestens eine Verbindung nach Anspruch 1-18 und/oder ihre pharmazeutisch verwendbaren Salze, Solvate und Stereoisomere, einschließlich deren Mischungen in allen Verhältnissen, sowie gegebenenfalls Träger- und/oder Hilfsstoffe.

21. Verwendung von Verbindungen gemäß Anspruch 1-18, sowie ihrer pharmazeutisch verwendbaren Salze, Solvate und Stereoisomere, einschließlich deren Mischungen in allen Verhältnissen, zur Herstellung eines Arzneimittels zur Behandlung und/oder Prophylaxe von Krankheiten, bei denen die Hemmung, Regulierung und/oder Modulation der Signaltransduktion von Kinasen eine Rolle spielt.

22. Verwendung nach Anspruch 21, wobei es sich bei der Kinase um SGK handelt.

23. Verwendung nach Anspruch 22 von Verbindungen gemäß Anspruch 1-18, sowie ihrer pharmazeutisch verwendbaren Salze, Solvate und Stereoisomere, einschließlich deren Mischungen in allen Verhältnissen, zur Herstellung eines Arzneimittels zur Behandlung von Krankheiten, die durch Inhibierung der SGK durch die Verbindungen nach Anspruch 1-18 beeinflußt werden.

24. Verwendung nach Anspruch 23 von Verbindungen gemäß Anspruch 1-18, sowie ihrer pharmazeutisch verwendbaren Salze, Solvate und Stereoisomere, einschließlich deren Mischungen in allen Verhältnissen, zur Herstellung eines Arzneimittels zur Behandlung oder Vorbeugung von Diabetes, Fettsucht, metabolischem Syndrom (Dyslipidämie), systemischer und pulmonaler Hypertonie, Herzkreislauferkrankungen und Nierenerkrankungen, allgemein bei jeglicher Art von Fibrosen und entzündlichen Prozessen, Krebs, Tumorzellen, Tumormetastasen, Koagulopathien, neuronaler Erregbarkeit, Glaukom, Katarakt, bakteriellen Infektionen sowie in einer antiinfektiösen Therapie, zur Steigerung der Lernfähigkeit und Aufmerksamkeit, sowie zur Behandlung und Prophylaxe von Zellalterung und Stress und zur Behandlung von Tinitus.

25. Verwendung nach Anspruch 24, wobei es sich bei Diabetes um Diabetes mellitus, diabetische Nephropathie, diabetische Neuropathie, diabetische Angiopathie und Mikroangiopathie handelt.

26. Verwendung nach Anspruch 24, wobei es sich bei Herzkreislauferkrankungen um kardiale Fibrosen nach Myokardinfarkt, Herzhypertrophie, Herzinsuffizienz und Arteriosklerose handelt.

27. Verwendung nach Anspruch 24, wobei es sich bei Nierenerkrankungen um Glomerulosklerose, Nephrosklerose, Nephritis, Nephropathie und Störung der Elektrolytausscheidung handelt.

28. Verwendung nach Anspruch 24, wobei es sich bei Fibrosen und entzündlichen Prozessen um Leberzirrhose, Lungenfibrose, fibrosierende Pankreatitis, Rheumatismus und Arthrosen, Morbus Crohn, chronische Bronchitis, Strahlenfibrose, Sklerodermitis, zystische Fibrose, Narbenbildung und Morbus Alzheimer handelt.

29. Arzneimittel enthaltend mindestens eine Verbindung gemäß Anspruch 1-18 und/oder ihre pharmazeutisch verwendbaren Salze, Solvate und Stereoisomere, einschließlich deren Mischungen in allen Verhältnissen, und mindestens einen weiteren Arzneimittelwirkstoff.

30. Set (Kit), bestehend aus getrennten Packungen von
(a) einer wirksamen Menge an einer Verbindung gemäß Anspruch 1-18 und/oder ihrer pharmazeutisch verwendbaren Salze, Solvate und Stereoisomere, einschließlich deren Mischungen in allen Verhältnissen,
und
(b) einer wirksamen Menge eines weiteren Arzneimittelswirkstoffs.

## Claims

1. Compounds of the formula I 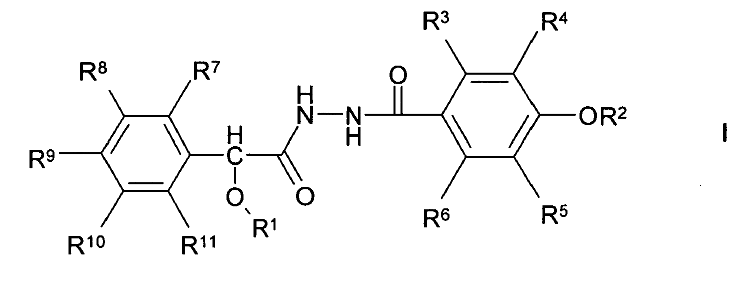 in which
R¹, R² each, independently of one another, denote H, CHO or acetyl,
R³, R⁴, R⁵, R⁶, R⁷,
R⁸, R⁹, R¹⁰, R¹¹ each, independently of one another, denote
H, A, OSO₂A, Hal, NO₂, OR¹², N(R¹²)₂, CN, O-COA, -[C(R¹²)₂]ₙCOOR¹², O-[C(R¹²)₂]ₒCOOR¹², SO₃H, -[C(R¹²)₂]ₙAr, -CO-Ar, O-[C(R¹²)₂]ₙAr, -[C(R¹²)₂]ₙHet, -[C(R¹²)₂]ₙC≡CH, O-[C(R¹²)₂]ₙC≡CH, -[C(R¹²)₂]ₙCON(R¹²)₂, -[C(R¹²)₂]ₙCONR¹²N(R¹²)₂, O-[C(R¹²)₂]ₙCON(R¹²)₂, O-[C(R¹²)₂]ₒCONR¹²N(R¹²)₂, NR¹²COA, NR¹²CON(R¹²)₂,
NR¹²SO₂A, N(SO₂A)₂, COR¹², S(O)ₘAr, SO₂NR¹² or S(O)ₘA, R³ and R⁴ together also denote CH=CH-CH=CH,
R³ and R⁴, R⁷ and R⁸
or R⁸ and R⁹ together also denote alkylene having 3, 4 or 5 C atoms,
in which one or two CH₂ groups may be replaced by oxygen,
A denotes unbranched or branched alkyl having 1-6 C atoms, in which 1-7 H atoms may be replaced by F,
or cyclic alkyl having 3-7 C atoms,
Ar denotes phenyl, naphthyl or biphenyl, each of which is unsubstituted or mono-, di- or trisubstituted by Hal, A, OR¹², N(R¹²)₂, NO₂, CN, phenyl, CON(R¹²)₂, NR¹²COA, NR¹²CON(R¹²)₂, NR¹²SO₂A, COR¹², SO₂N(R¹²)₂, S(O)ₘA, -[C(R¹²)₂]ₙ-COOR¹² and/or -O[C(R¹²)₂]ₒ-COOR¹²,
Het denotes a mono- or bicyclic saturated, unsaturated or aromatic heterocycle having 1 to 4 N, O and/or S atoms, which may be mono-, di- or trisubstituted by Hal, A, OR¹², N(R¹²)₂, NO₂, CN, COOR¹², CON(R¹²)₂, NR¹²COA, NR¹²SO₂A, COR¹², SO₂NR¹², S(O)mA, =S, =NR¹² and/or =O (carbonyl oxygen),
R¹² denotes H or A,
Hal denotes F, Cl, Br or I,
m denotes 0, 1 or 2,
n denotes 0, 1, 2 or 3,
o denotes 1, 2 or 3,
and pharmaceutically usable salts, solvates and stereoisomers thereof, including mixtures thereof in all ratios.

2. Compounds according to Claim 1 in which
R¹ denotes H or CHO,
R² denotes H,
and pharmaceutically usable salts, solvates and stereoisomers thereof, including mixtures thereof in all ratios.

3. Compounds according to Claim 1 or 2 in which R³, R⁴, R⁵, R⁶, R⁷,
R⁸, R⁹, R¹⁰, R¹¹ each, independently of one another, denote
H, A, Hal, OR¹² or O-[C(R¹²)₂]ₙAr,
and pharmaceutically usable salts, solvates and stereoisomers thereof, including mixtures thereof in all ratios.

4. Compounds according to one or more of Claims 1-3 in which R⁶ denotes OH,
and pharmaceutically usable salts, solvates and stereoisomers thereof, including mixtures thereof in all ratios.

5. Compounds according to one or more of Claims 1-4 in which R³ denotes H, A or Hal,
and pharmaceutically usable salts, solvates and stereoisomers thereof, including mixtures thereof in all ratios.

6. Compounds according to one or more of Claims 1-5 in which R⁸ denotes OH, A, phenoxy or Hal,
and pharmaceutically usable salts, solvates and stereoisomers thereof, including mixtures thereof in all ratios.

7. Compounds according to one or more of Claims 1-6 in which R⁴, R⁵, R⁷, R⁹, R¹⁰, R¹¹ denote H or A,
and pharmaceutically usable salts, solvates and stereoisomers thereof, including mixtures thereof in all ratios.

8. Compounds according to one or more of Claims 1-7 in which R⁷, R¹⁰, R¹¹ each, independently of one another, denote H or Hal, and pharmaceutically usable salts, solvates and stereoisomers thereof, including mixtures thereof in all ratios.

9. Compounds according to one or more of Claims 1-8 in which
Ar denotes phenyl which is unsubstituted or mono-, di- or trisubstituted by Hal and/or A,
and pharmaceutically usable salts, solvates and stereoisomers thereof, including mixtures thereof in all ratios.

10. Compounds according to one or more of Claims 1-9 in which Ar denotes phenyl,
and pharmaceutically usable salts, solvates and stereoisomers thereof, including mixtures thereof in all ratios.

11. Compounds according to one or more of Claims 1-10 in which
Het denotes a monocyclic saturated, unsaturated or aromatic heterocycle having 1 to 2 N and/or O atoms, which may be unsubstituted or mono-, di- or trisubstituted by A, Hal, OH and/or OA,
and pharmaceutically usable salts, solvates and stereoisomers thereof, including mixtures thereof in all ratios.

12. Compounds according to one or more of Claims 1-11 in which Het denotes a monocyclic saturated heterocycle having 1 to 2 N
and/or O atoms, which may be unsubstituted or mono- or disubstituted by A,
and pharmaceutically usable salts, solvates and stereoisomers thereof, including mixtures thereof in all ratios.

13. Compounds according to one or more of Claims 1-12 in which Het denotes furyl, thienyl, pyrrolyl, imidazolyl, pyridyl, pyrimidinyl,
pyrazolyl, thiazolyl, indolyl, pyrrolidinyl, piperidinyl, morpholinyl or piperazinyl, each of which is unsubstituted or mono-, di- or trisubstituted by A, Hal, OH and/or OA,
and pharmaceutically usable salts, solvates and stereoisomers thereof, including mixtures thereof in all ratios.

14. Compounds according to one or more of Claims 1-13 in which R¹ denotes H or CHO,
R² denotes H,
R³, R⁴, R⁵, R⁶, R⁷,
R⁸, R⁹, R¹⁰, R¹¹ each, independently of one another, denote
H, A, Hal, OR¹² or O-[C(R¹²)₂]ₙAr,
and pharmaceutically usable salts, solvates and stereoisomers thereof, including mixtures thereof in all ratios.

15. Compounds according to one or more of Claims 1-14 in which R¹ denotes H or CHO,
R² denotes H,
R³ denotes H, A or Hal,
R⁴, R⁵, R⁷, R⁹, R¹⁰, R¹¹ denote H or A,
R⁶ denotes OH,
R⁸ denotes OH, A, phenoxy or Hal,
and pharmaceutically usable salts, solvates and stereoisomers thereof, including mixtures thereof in all ratios.

16. Compounds according to one or more of Claims 1-15 in which R¹ denotes H or CHO,
R² denotes H,
R³ denotes H, A or Hal,
R⁴, R⁵, R⁷, R⁹, R¹⁰, R¹¹ denote H, A or Hal,
R⁸ and R⁹ together also denote methylenedioxy,
R⁶ denotes OH,
R⁸ denotes OH, A, phenoxy or Hal,
and pharmaceutically usable salts, solvates and stereoisomers thereof, including mixtures thereof in all ratios.

17. Compounds according to one or more of Claims 1-16 in which R¹ denotes H, CHO or acetyl,
R² denotes H,
R³ denotes H, A or Hal,
R⁴, R⁵, R⁷,
R¹⁰, R¹¹ each, independently of one another, denote H, A or Hal, R⁶ denotes OH,
R⁸ denotes OH, A, phenoxy or Hal,
R⁹ denotes H, Hal or OA,
R⁸ and R⁹ together also denote methylenedioxy,
and pharmaceutically usable salts, solvates and stereoisomers thereof, including mixtures thereof in all ratios.

18. Compounds according to Claim 1 selected from the group
| No. | Structural formula Name |
|---|---|
| "A1" | 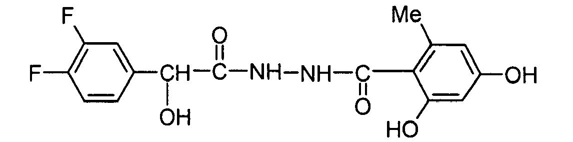 |
| | *N'*-[2-(3,4-Difluorophenyl)-2-hydroxyacetyl]-2,4-dihydroxy-6-methylbenzohydrazide |
| "A2" | 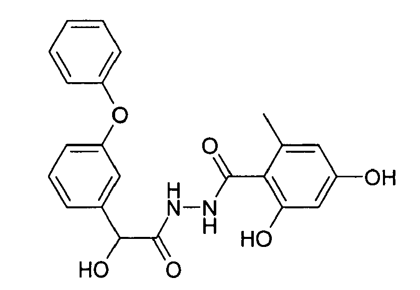 |
| | *N*'-[2-Hydroxy-2-(3-phenoxyphenyl)acetyl]-2,4-dihydroxy-6-methylbenzohydrazide |
| "A3" | 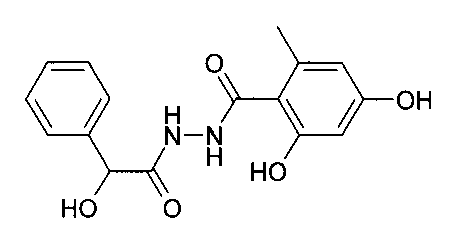 |
| | *N*'-(2-Hydroxy-2-phenylacetyl)-2,4-dihydroxy-6-methyl-benzohydrazide |
| "A4" | 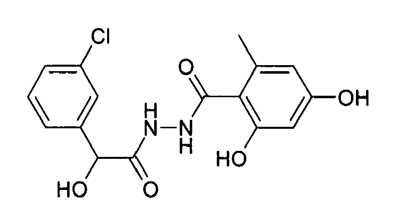 |
| | *N*'-[2-Hydroxy-2-(3-chlorophenyl)acetyl]-2,4-dihydroxy-6-methylbenzohydrazide |
| "A5" | 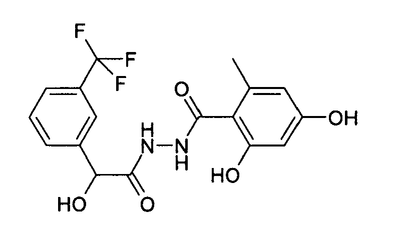 |
| | *N*'-[2-Hydroxy-2-(3-trifluoromethylphenyl)acetyl]-2,4-dihydroxy-6-methylbenzohydrazide |
| "A6" | 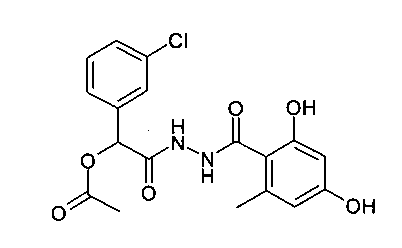 |
| | *N*'-[2-Acetoxy-2-(3-chlorophenyl)acetyl]-2,4-dihydroxy-6-methylbenzohydrazide |
| "A7" | 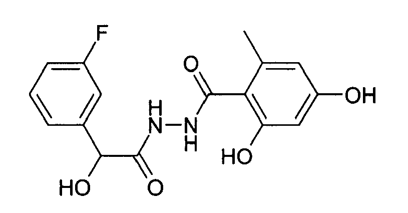 |
| | *N*'-[2-Hydroxy-2-(3-fluorophenyl)acetyl]-2,4-dihydroxy-6-methylbenzohydrazide |
| "A8" | 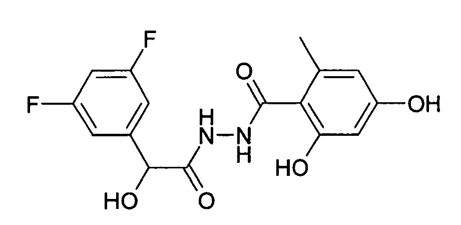 |
| | *N*'-[2-Hydroxy-2-(3,5-difluorophenyl)acetyl]-2,4-dihydroxy-6-methylbenzohydrazide |
| "A9" | 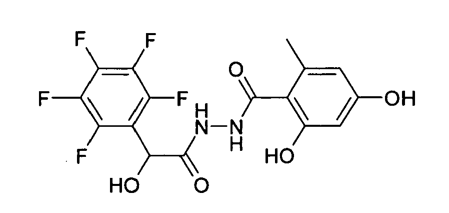 |
| | *N*'-[2-Hydroxy-2-(2,3,4,5,6-pentafluorophenyl)acetyl]-2,4-dihydroxy-6-methylbenzohydrazide |
| "A10" | 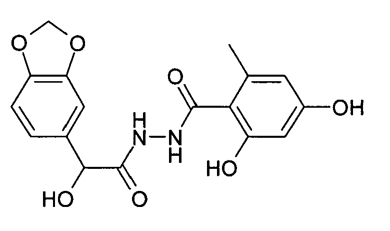 |
| | *N*'-(2-Benzo-1,3-dioxol-5-yl-2-hydroxyacetyl)-2,4-dihydroxy-6-methylbenzohydrazide |
| "A11" | 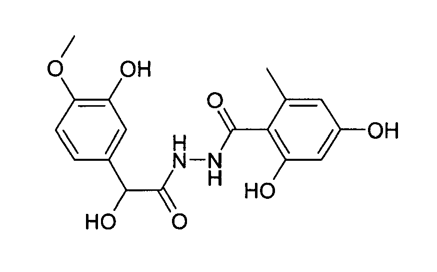 |
| | *N*'-[2-Hydroxy-2-(3-hydroxy-4-methoxyphenyl)acetyl]-2,4-dihydroxy-6-methylbenzohydrazide |
| "A12" | 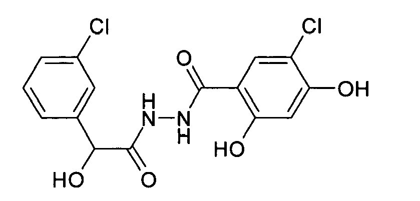 |
| | *N*'-[2-Hydroxy-2-(3-chlorophenyl)acetyl]-2,4-dihydroxy-5-chlorobenzohydrazide |
| "A13" | 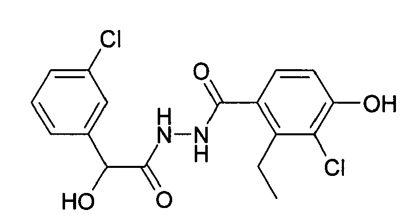 |
| | *N*'-[2-Hydroxy-2-(3-chlorophenyl)acetyl]-3-chloro-2-ethyl-4-hydroxybenzohydrazide |
| "A14" | 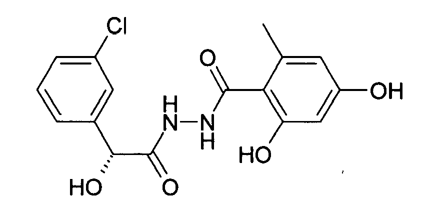 |
| | *N*'-[(R)-2-(3-Chlorophenyl)-2-hydroxyacetyl]-2,4-dihydroxy-6-methylbenzohydrazide |
| "A15" | 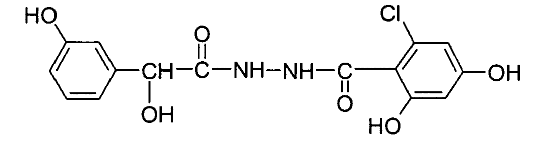 |
| | *N*'-[2-Hydroxy-2-(3-hydroxyphenyl)acetyl]-2-chloro-4,6-dihydroxybenzohydrazide |
| "A16" | 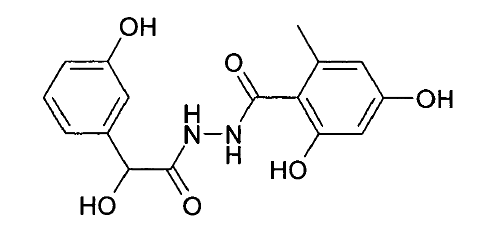 |
| | *N*'-[2-Hydroxy-2-(3-hydroxyphenyl)acetyl]-2-methyl-4,6-dihydroxybenzohydrazide |
| "A17" | 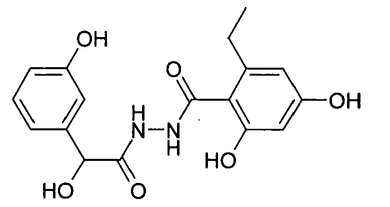 |
| | *N*'-[2-Hydroxy-2-(3-hydroxyphenyl)acetyl]-2-ethyl-4,6-dihydroxybenzohydrazide |
| "A18" | 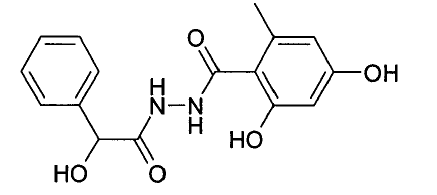 |
| | *N*'-(2-Hydroxy-2-phenylacetyl)-2-methyl-4,6-dihydroxybenzohydrazide |
| "A19" | 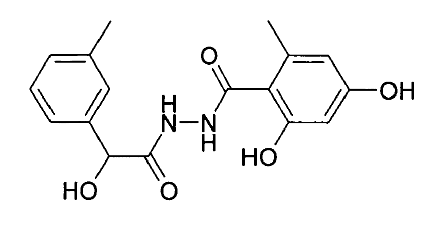 |
| | *N*'-[2-Hydroxy-2-(3-methylphenyl)acetyl]-2,4-dihydroxy-6-methylbenzohydrazide |
| "A20" | 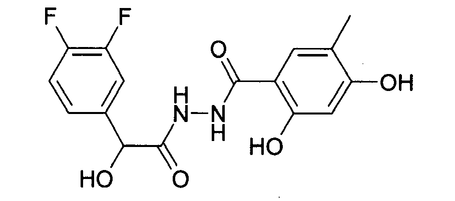 |
| | *N*'-[2-Hydroxy-2-(3,4-difluorophenyl)acetyl]-2,4-dihydroxy-5-methylbenzohydrazide |
| "A21" | 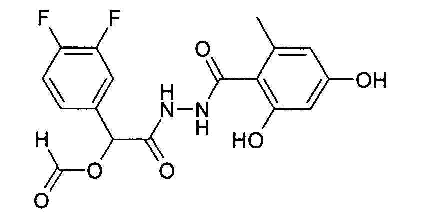 |
| | *N*'-[2-Formyloxy-2-(3,4-difluorophenyl)acetyl]-2,4-dihydroxy-6-methylbenzohydrazide |
| "A22" | 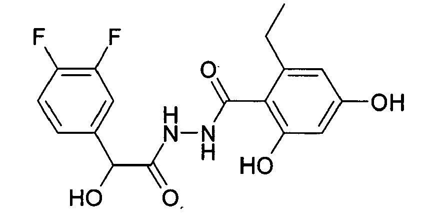 |
| | *N*'-[2-Hydroxy-2-(3,4-difluorophenyl)acetyl]-2,4-dihydroxy-6-ethylbenzohydrazide |
| "A23" | 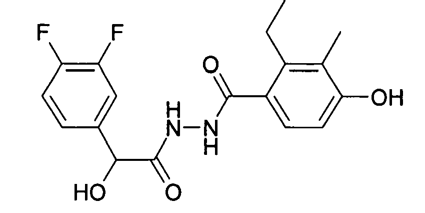 |
| | *N*'-[2-Hydroxy-2-(3,4-difluorophenyl)acetyl]-4-hydroxy-2-ethyl-3-methylbenzohydrazide |
and pharmaceutically usable salts, solvates and stereoisomers thereof, including mixtures thereof in all ratios.

19. Process for the preparation of compounds of the formula I according to Claims 1-18 and pharmaceutically usable solvates, salts and stereoisomers thereof, **characterised in that**
a) a compound of the formula II 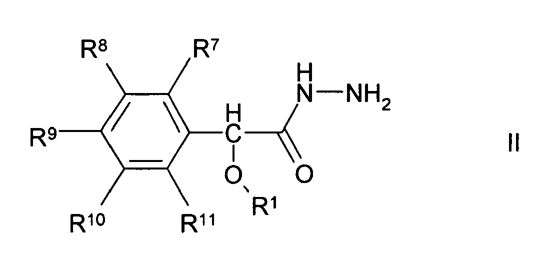 in which
R¹, R⁷, R⁸, R⁹, R¹⁰ and R¹¹ have the meanings indicated in Claim 1,
is reacted with a compound of the formula III 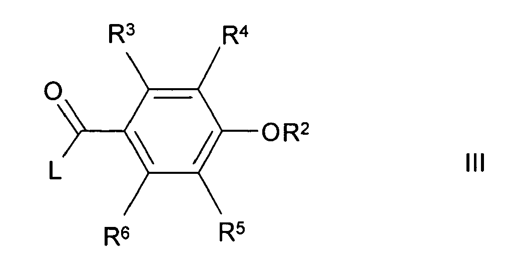 in which
L denotes Cl, Br, I or a free or reactively functionally modified OH group and
R², R³, R⁴, R⁵ and R⁶ have the meanings indicated in Claim 1,
or
b) a compound of the formula IV 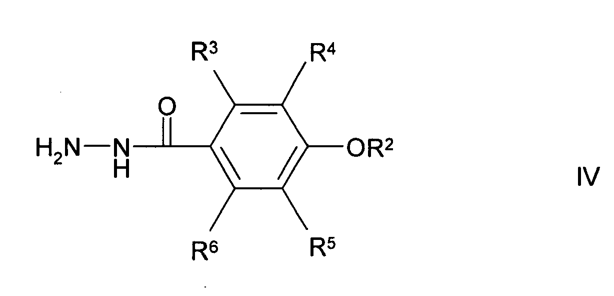 in which
R², R³, R⁴, R⁵ and R⁶ have the meanings indicated in Claim 1,
is reacted with a compound of the formula V 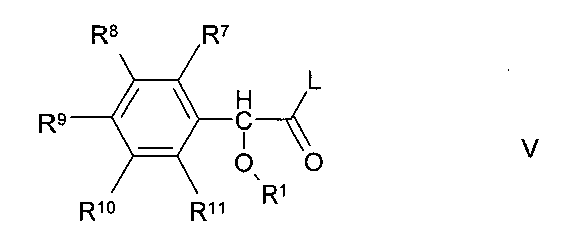 in which
L denotes Cl, Br, I or a free or reactively functionally modified OH group and
R¹, R⁷, R⁸, R⁹, R¹⁰ and R¹¹ have the meanings indicated in Claim 1,
or
c) a radical R¹, R², R³, R⁴, R⁵, R⁶, R⁷, R⁸, R⁹, R¹⁰ and/or R¹¹ in a compound of the formula I is converted into another radical R¹, R², R³, R⁴, R⁵, R⁶, R⁷, R⁸, R⁹, R¹⁰, and/or R¹¹
by cleaving an ether by hydrolysis or hydrogenolysis,
and/or a base or acid of the formula I is converted into one of its salts.

20. Medicaments comprising at least one compound according to Claims 1-18 and/or pharmaceutically usable salts, solvates and stereoisomers thereof, including mixtures thereof in all ratios, and optionally excipients and/or adjuvants.

21. Use of compounds according to Claims 1-18, and pharmaceutically usable salts, solvates and stereoisomers thereof, including mixtures thereof in all ratios, for the preparation of a medicament for the treatment and/or prophylaxis of diseases in which the inhibition,
regulation and/or modulation of kinase signal transduction plays a role.

22. Use according to Claim 21, where the kinase is SGK.

23. Use according to Claim 22 of compounds according to Claims 1-18, and pharmaceutically usable salts, solvates and stereoisomers thereof, including mixtures thereof in all ratios, for the preparation of a medicament for the treatment of diseases which are influenced by inhibition of SGKs by the compounds according to Claims 1-18.

24. Use according to Claim 23 of compounds according to Claims 1-18, and pharmaceutically usable salts, solvates and stereoisomers thereof, including mixtures thereof in all ratios, for the preparation of a medicament for the treatment or prevention of diabetes, obesity, metabolic syndrome (dyslipidaemia), systemic and pulmonary hypertonia, cardiovascular diseases and kidney diseases, generally in fibroses and inflammatory processes of any type, cancer, tumour cells, tumour metastases, coagulopathies, neuronal excitability, glaucoma, cataract, bacterial infections and in antiinfection therapy, for increasing learning ability and attention, and for the treatment and prophylaxis of cell ageing and stress, and for the treatment of tinnitus.

25. Use according to Claim 24, where diabetes is diabetes mellitus, diabetic nephropathy, diabetic neuropathy, diabetic angiopathy and microangiopathy.

26. Use according to Claim 24, where cardiovascular diseases are cardiac fibroses after myocardial infarction, cardiac hypertrophy, cardiac insufficiency and arteriosclerosis.

27. Use according to Claim 24, where kidney diseases are glomerulosclerosis, nephrosclerosis, nephritis, nephropathy and electrolyte excretion disorder.

28. Use according to Claim 24, where fibroses and inflammatory processes are liver cirrhosis, pulmonary fibrosis, fibrosing pancreatitis, rheumatism and arthroses, Crohn's disease, chronic bronchitis, radiation fibrosis, sclerodermatitis, cystic fibrosis, scarring and Alzheimer's disease.

29. Medicaments comprising at least one compound according to Claims 1-18 and/or pharmaceutically usable salts, solvates and stereoisomers thereof, including mixtures thereof in all ratios, and at least one further medicament active compound.

30. Set (kit) consisting of separate packs of
(a) an effective amount of a compound according to Claims 1-18 and/or pharmaceutically usable salts, solvates and stereoisomers thereof, including mixtures thereof in all ratios,
and
(b) an effective amount of a further medicament active compound.

## Revendications

1. Composés de formule I 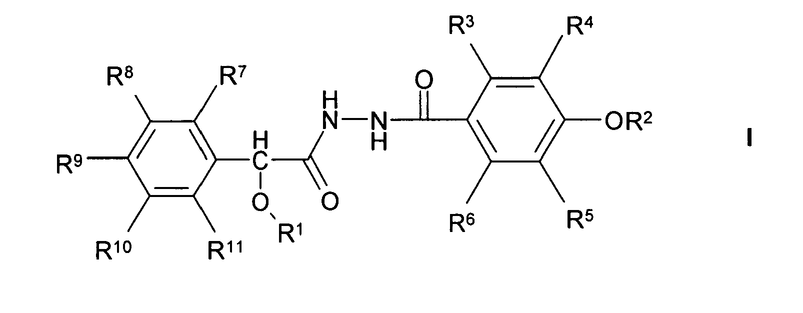 dans laquelle
R¹, R² désignent chacun, indépendamment l'un de l'autre, H, CHO
ou acétyle,
R³, R⁴, R⁵, R⁶, R⁷,
R⁸, R⁹, R¹⁰, R¹¹ désignent chacun, indépendamment l'un de l'autre,
H, A, OSO₂A, Hal, NO₂, OR¹², N(R¹²)₂, CN, O-COA, -[C(R¹²)₂]ₙCOOR¹², O-[C(R¹²)₂]ₒCOOR¹², SO₃H, -[C(R¹²)₂]ₙAr, -CO-Ar, O-[C(R¹²)₂]ₙAr, -[C(R¹²)₂]ₙHét, -[C(R¹²)₂]ₙC≡CH, O-[C(R¹²)₂]ₙC≡CH, -[C(R¹²)₂]ₙCON(R¹²)₂, -[C(R¹²)₂]ₙCONR¹²N(R¹²)₂, O-[C(R¹²)₂]ₙCON(R¹²)₂, O-[C(R¹²)₂]ₒCONR¹²N(R¹²)₂, NR¹²COA, NR¹²CON(R¹²)₂, NR¹²SO₂A, N(SO₂A)₂, COR¹², S(O)ₘAr, SO₂NR¹² ou S(O)ₘA,
R³ et R⁴ désignent aussi ensemble CH=CH-CH=CH,
R³ et R⁴, R⁷ et R⁸
ou R⁸ et R⁹ désignent aussi ensemble alkylène ayant 3, 4 ou 5
atomes de C,
où un ou deux groupements CH₂ peuvent être remplacés par oxygène,
A désigne alkyle non ramifié ou ramifié ayant 1-6 atomes de C,
où 1-7 atomes de H peuvent être remplacés par F,
ou alkyle cyclique ayant 3-7 atomes de C,
Ar désigne phényle, naphtyle ou biphényle, chacun d'entre eux
étant non substitué ou mono-, di- ou trisubstitué par Hal, A, OR¹², N(R¹²)₂, NO₂, CN, phényle, CON(R¹²)₂, NR¹²COA, NR¹²CON(R¹²)₂, NR¹²SO₂A, COR¹², SO₂N(R¹²)₂, S(O)ₘA, -[C(R¹²)₂]ₙ-COOR¹² et/ou -O[C(R¹²)₂]ₒ-COOR¹²,
Hét désigne un hétérocycle mono- ou bicyclique saturé, insaturé
ou aromatique ayant 1 à 4 atomes de N, O et/ou S, pouvant être mono-, di- ou trisubstitué par Hal, A, OR¹², N(R¹²)₂, NO₂, CN, COOR¹², CON(R¹²)₂, NR¹²COA, NR¹²SO₂A, COR¹², SO₂NR¹², S(O)ₘA, =S, =NR¹² et/ou =O (oxygène carbonyle), R¹² désigne H ou A,
Hal désigne F, CI, Br ou I,
m désigne 0, 1 ou 2,
n désigne 0, 1, 2 ou 3,
o désigne 1, 2 ou 3,
et les sels, solvats et stéréoisomères pharmaceutiquement utilisables de ceux-ci, y compris des mélanges de ceux-ci en toutes proportions.

2. Composés selon la revendication 1, dans lesquels
R¹ désigne H ou CHO,
R² désigne H,
et les sels, solvats et stéréoisomères pharmaceutiquement utilisables de ceux-ci, y compris des mélanges de ceux-ci en toutes proportions.

3. Composés selon la revendication 1 ou 2, dans lesquels
R³, R⁴, R⁵, R⁶, R⁷,
R⁸, R⁹, R¹⁰, R¹¹ désignent chacun, indépendamment l'un de l'autre,
H, A, Hal, OR¹² ou O-[C(R¹²)₂]ₙAr,
et les sels, solvats et stéréoisomères pharmaceutiquement utilisables de ceux-ci, y compris des mélanges de ceux-ci en toutes proportions.

4. Composés selon l'une ou plusieurs des revendications 1-3, dans lesquels
R⁶ désigne OH,
et les sels, solvats et stéréoisomères pharmaceutiquement utilisables de ceux-ci, y compris des mélanges de ceux-ci en toutes proportions.

5. Composés selon l'une ou plusieurs des revendications 1-4, dans lesquels
R³ désigne H, A ou Hal,
et les sels, solvats et stéréoisomères pharmaceutiquement utilisables de ceux-ci, y compris des mélanges de ceux-ci en toutes proportions.

6. Composés selon l'une ou plusieurs des revendications 1-5, dans lesquels
R⁸ désigne OH, A, phénoxy ou Hal,
et les sels, solvats et stéréoisomères pharmaceutiquement utilisables de ceux-ci, y compris des mélanges de ceux-ci en toutes proportions.

7. Composés selon l'une ou plusieurs des revendications 1-6, dans lesquels
R⁴, R⁵, R⁷, R⁹, R¹⁰, R¹¹ désignent H ou A,
et les sels, solvats et stéréoisomères pharmaceutiquement utilisables de ceux-ci, y compris des mélanges de ceux-ci en toutes proportions.

8. Composés selon l'une ou plusieurs des revendications 1-7, dans lesquels
R⁷, R¹⁰, R¹¹ désignent chacun, indépendamment l'un de l'autre, H ou Hal,
et les sels, solvats et stéréoisomères pharmaceutiquement utilisables de ceux-ci, y compris des mélanges de ceux-ci en toutes proportions.

9. Composés selon l'une ou plusieurs des revendications 1-8, dans lesquels
Ar désigne phényle qui est non substitué ou mono-, di- ou trisubstitué par Hal et/ou A,
et les sels, solvats et stéréoisomères pharmaceutiquement utilisables de ceux-ci, y compris des mélanges de ceux-ci en toutes proportions.

10. Composés selon l'une ou plusieurs des revendications 1-9, dans lesquels
Ar désigne phényle,
et les sels, solvats et stéréoisomères pharmaceutiquement utilisables de ceux-ci, y compris des mélanges de ceux-ci en toutes proportions.

11. Composés selon l'une ou plusieurs des revendications 1-10, dans lesquels
Hét désigne un hétérocycle monocyclique saturé, insaturé ou aromatique ayant 1 à 2 atomes de N et/ou O, pouvant être non substitué ou mono-, di- ou trisubstitué par A, Hal, OH et/ou OA,
et les sels, solvats et stéréoisomères pharmaceutiquement utilisables de ceux-ci, y compris des mélanges de ceux-ci en toutes proportions.

12. Composés selon l'une ou plusieurs des revendications 1-11, dans lesquels
Hét désigne un hétérocycle monocyclique saturé ayant 1 à 2 atomes de N et/ou O, pouvant être non substitué ou mono-ou disubstitué par A,
et les sels, solvats et stéréoisomères pharmaceutiquement utilisables de ceux-ci, y compris des mélanges de ceux-ci en toutes proportions.

13. Composés selon l'une ou plusieurs des revendications 1-12, dans lesquels
Hét désigne furyle, thiényle, pyrrolyle, imidazolyle, pyridyle, pyrimidinyle, pyrazolyle, thiazolyle, indolyle, pyrrolidinyle, pipéridinyle, morpholinyle ou pipérazinyle, chacun d'entre eux étant non substitué ou mono-, di- ou trisubstitué par A, Hal, OH et/ou OA,
et les sels, solvats et stéréoisomères pharmaceutiquement utilisables de ceux-ci, y compris des mélanges de ceux-ci en toutes proportions.

14. Composés selon l'une ou plusieurs des revendications 1-13, dans lesquels
R¹ désigne H ou CHO,
R² désigne H,
R³ R⁴, R⁵, R⁶, R⁷,
R⁸, R⁹, R¹⁰, R¹¹ désignent chacun, indépendamment l'un de l'autre, H, A, Hal, OR¹² ou O-(C(R¹²)₂]ₙAr,
et les sels, solvats et stéréoisomères pharmaceutiquement utilisables de ceux-ci, y compris des mélanges de ceux-ci en toutes proportions.

15. Composés selon l'une ou plusieurs des revendications 1-14, dans lesquels
R¹ désigne H ou CHO,
R² désigne H,
R³ désigne H, A ou Hal,
R⁴, R⁵, R⁷, R⁹, R¹⁰, R¹¹ désignent H ou A,
R⁶ désigne OH,
R⁸ désigne OH, A, phénoxy ou Hal,
et les sels, solvats et stéréoisomères pharmaceutiquement utilisables de ceux-ci, y compris des mélanges de ceux-ci en toutes proportions.

16. Composés selon l'une ou plusieurs des revendications 1-15, dans lesquels
R¹ désigne H ou CHO,
R² désigne H,
R³ désigne H, A ou Hal,
R⁴, R⁵, R⁷, R⁹, R¹⁰, R¹¹ désignent H, A ou Hal,
R⁸ et R⁹ désignent aussi ensemble méthylènedioxy,
R⁶ désigne OH,
R⁸ désigne OH, A, phénoxy ou Hal,
et les sels, solvats et stéréoisomères pharmaceutiquement utilisables de ceux-ci, y compris des mélanges de ceux-ci en toutes proportions.

17. Composés selon l'une ou plusieurs des revendications 1-16, dans lesquels
R¹ désigne H, CHO ou acétyle,
R² désigne H,
R³ désigne H, A ou Hal,
R⁴, R⁵, R⁷,
R¹⁰, R¹¹ désignent chacun, indépendamment l'un de l'autre, H, A ou Hal,
R⁶ désigne OH,
R⁸ désigne OH, A, phénoxy ou Hal,
R⁹ désigne H, Hal ou OA,
R⁸ et R⁹ désignent aussi ensemble méthylènedioxy,
et les sels, solvats et stéréoisomères pharmaceutiquement utilisables de ceux-ci, y compris des mélanges de ceux-ci en toutes proportions.

18. Composés selon la revendication 1, choisis dans le groupe constitué par
| n° | Formule structurelle Nom |
|---|---|
| "A1" | 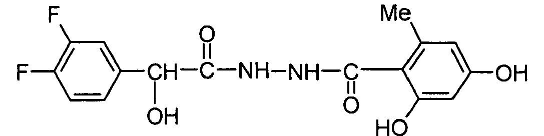 |
| | *N'*-(2-(3,4-Difluorophényl)-2-hydroxyacétyl]-2,4-dihydroxy-6-méthylbenzohydrazide |
| "A2" | 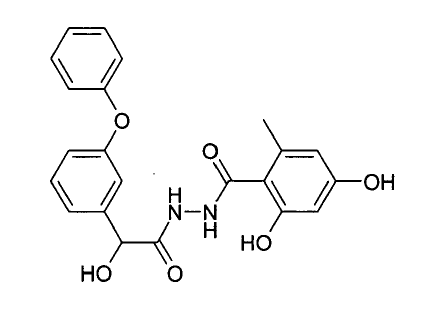 |
| | *N'*-[2-Hydroxy-2-(3-phénoxyphényl)acétyl]-2,4-dihydroxy-6-méthylbenzohydrazide |
| "A3" | 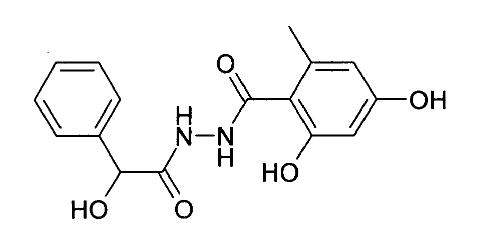 |
| | *N'*-(2-Hydroxy-2-phénylacétyl)-2,4-dihydroxy-6-méthyl-benzohydrazide |
| "A4" | 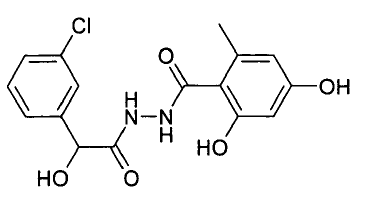 |
| | *N'*-[2-Hydroxy-2-(3-chlorophényl)acétyl]-2,4-dihydroxy-6-méthylbenzohydrazide |
| "A5" | 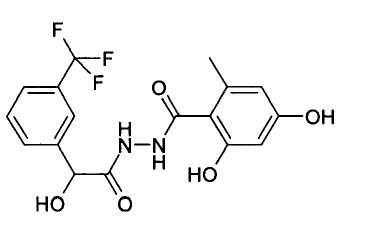 |
| | *N'*-[2-Hydroxy-2-(3-trifluorométhylphényl)acétyl]-2,4-dihydroxy-6-méthylbenzohydrazide |
| "A6" | 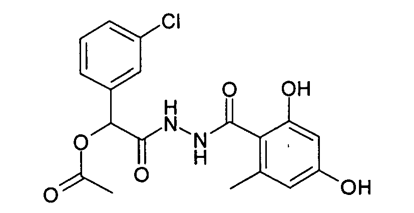 |
| | *N'*-[2-Acétoxy-2-(3-chlorophényl)acétyl]-2,4-dihydroxy-6-méthylbenzohydrazide |
| "A7" | 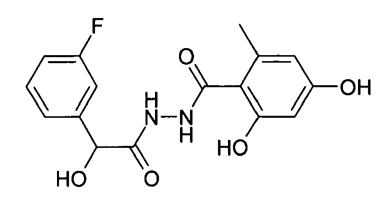 |
| | *N'*-[2-Hydroxy-2-(3-fluorophényl)acétyl]-2,4-dihydroxy-6-méthylbenzohydrazide |
| "A8" | 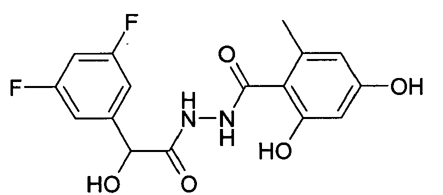 |
| | *N'*-[2-Hydroxy-2-(3,5-difluorophényl)acétyl]-2,4-dihydroxy-6-méthylbenzohydrazide |
| "A9" | 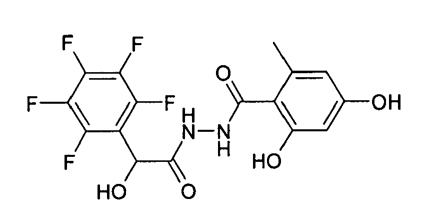 |
| | *N'*-[2-Hydroxy-2-(2,3,4,5,6-pentafluorophényl)acétyl]-2,4-dihydroxy-6-méthylbenzohydrazide |
| "A10" | 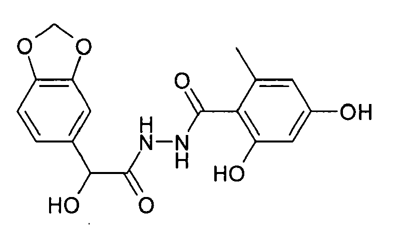 |
| | *N'*-(2-Benzo-1,3-dioxol-5-yl-2-hydroxyacétyl)-2,4-dihydroxy-6-méthylbenzohydrazide |
| "A11" | 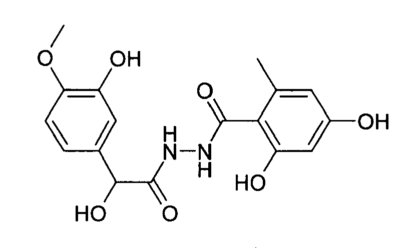 |
| | *N'*-[2-Hydroxy-2-(3-hydroxy-4-méthoxyphényl)acétyl]-2,4-dihydroxy-6-méthylbenzohydrazide |
| "A12" | 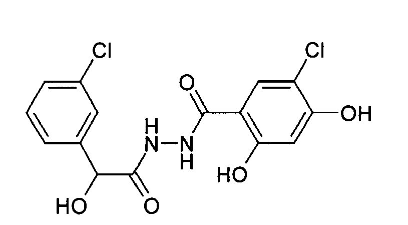 |
| | *N'*-[2-Hydroxy-2-(3-chlorophényl)acétyl]-2,4-dihydroxy-5-chlorobenzohydrazide |
| "A13" | 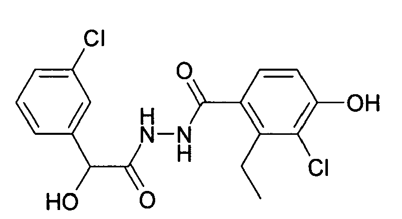 |
| | *N'*-[2-Hydroxy-2-(3-chlorophényl)acétyl]-3-chloro-2-éthyl-4-hydroxybenzohydrazide |
| "A14" | 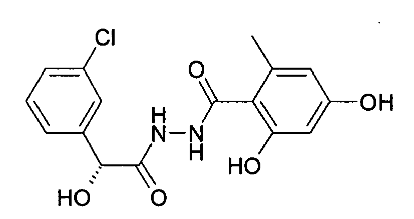 |
| | *N'*-[(R)-2-(3-Chlorophényl)-2-hydroxyacétyl]-2,4-dihydroxy-6-méthylbenzohydrazide |
| "A15" | 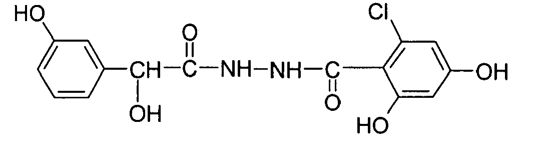 |
| | *N'*-[2-Hydroxy-2-(3-hydroxyphényl)acétyl]-2-chloro-4,6-dihydroxybenzohydrazide |
| "A16" | 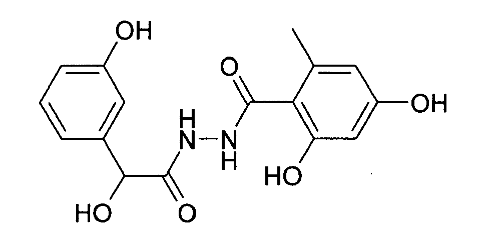 |
| | *N'*-[2-Hydroxy-2-(3-hydroxyphényl)acétyl]-2-méthyl-4,6-dihydroxybenzohydrazide |
| "A17" | 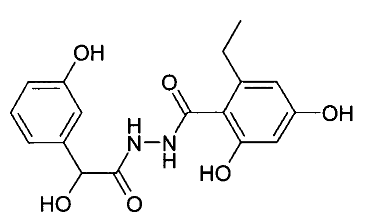 |
| | *N'*-[2-Hydroxy-2-(3-hydroxyphényl)acétyl]-2-éthyl-4,6-dihydroxybenzohydrazide |
| "A18" | 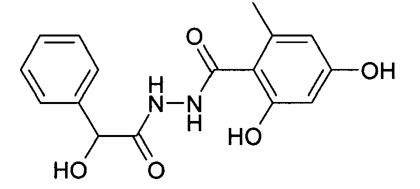 |
| | *N'*-(2-Hydroxy-2-phénylacétyl)-2-méthyl-4,6-dihydroxybenzohydrazide |
| "A19" | 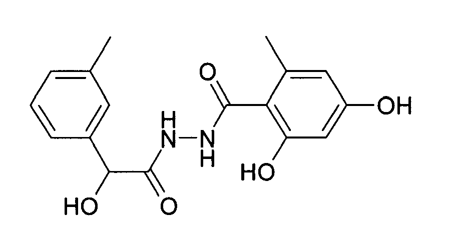 |
| | *N'*-[2-Hydroxy-2-(3-méthylphényl)acétyl]-2,4-dihydroxy-6-méthylbenzohydrazide |
| "A20" | 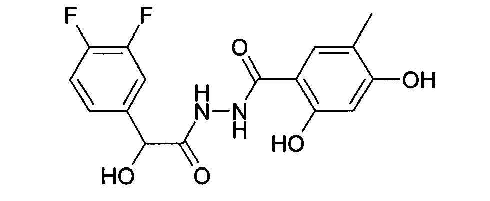 |
| | *N'*-[2-Hydroxy-2-(3,4-difluorophényl)acétyl]-2,4-dihydroxy-5-méthylbenzohydrazide |
| "A21" | 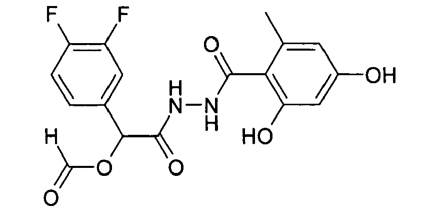 |
| | *N'*-[2-Formyloxy-2-(3,4-difluorophényl)acétyl]-2,4-dihydroxy-6-méthylbenzohydrazide |
| "A22" | 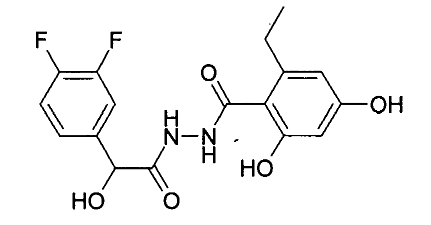 |
| | *N'*-[2-Hydroxy-2-(3,4-difluorophényl)acétyl]-2,4-dihydroxy-6-éthylbenzohydrazide |
| "A23" | 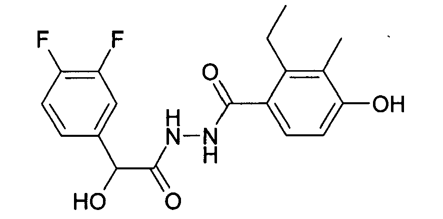 |
| | *N'*-[2-Hydroxy-2-(3,4-difluorophényl)acétyl]-4-hydroxy-2-éthyl-3-méthylbenzohydrazide |
et les sels, solvats et stéréoisomères pharmaceutiquement utilisables de ceux-ci, y compris des mélanges de ceux-ci en toutes proportions.

19. Procédé de préparation de composés de formule I selon les revendications 1-18 et de solvats, sels et stéréoisomères pharmaceutiquement utilisables de ceux-ci, **caractérisé en ce que**
a) un composé de formule II 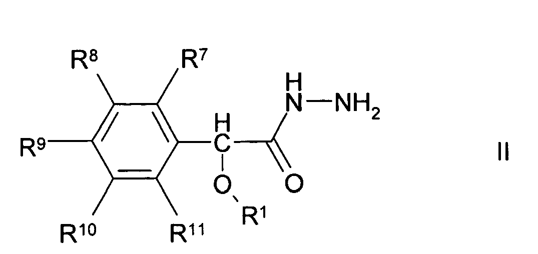 dans laquelle
R¹, R⁷, R⁸, R⁹, R¹⁰ et R¹¹ revêtent les significations indiquées selon la revendication 1,
est réagi avec un composé de formule III 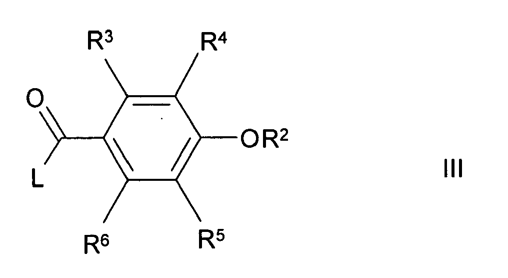 dans laquelle
L désigne Cl, Br, I ou un groupement OH libre ou modifié de manière réactive et fonctionnelle et
R², R³, R⁴, R⁵ et R⁶ revêtent les significations indiquées selon la revendication 1,
ou
b) un composé de formule IV 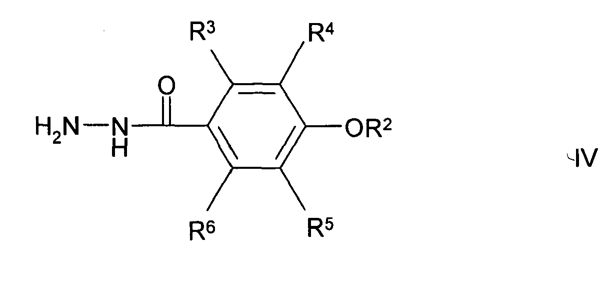 dans laquelle
R², R³, R⁴, R⁵ et R⁶ revêtent les significations indiquées selon la revendication 1,
est réagi avec un composé de formule V 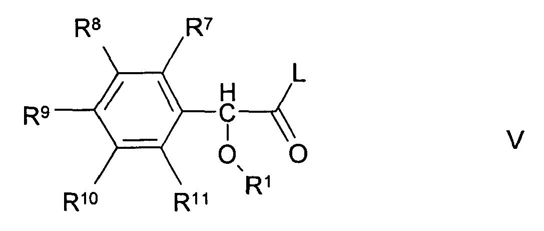 dans laquelle
L désigne Cl, Br, I ou un groupement OH libre ou modifié de manière réactive et fonctionnelle et
R¹, R⁷, R⁸, R⁹, R¹⁰ et R¹¹ revêtent les significations indiquées selon la revendication 1,
ou
c) un radical R¹, R², R³, R⁴, R⁵, R⁶, R⁷, R⁸, R⁹, R¹⁰ et/ou R¹¹ dans un composé de formule I est converti en un autre radical R¹, R², R³, R⁴, R⁵, R⁶, R⁷, R⁸, R⁹, R¹⁰ et/ou R¹¹
par le clivage d'un éther par hydrolyse ou hydrogénolyse,
et/ou une base ou un acide de formule I est converti(e) en l'un de ses sels.

20. Médicaments comprenant au moins un composé selon les revendications 1-18 et/ou des sels, solvats et stéréoisomères pharmaceutiquement utilisables de celui-ci, y compris des mélanges de ceux-ci en toutes proportions, et éventuellement des excipients et/ou des adjuvants.

21. Utilisation de composés selon les revendications 1-18, et de sels, solvats et stéréoisomères pharmaceutiquement utilisables de ceux-ci, y compris des mélanges de ceux-ci en toutes proportions, pour la préparation d'un médicament destiné au traitement et/ou à la prophylaxie de maladies dans lesquelles l'inhibition, la régulation et/ou la modulation de la transduction des signaux de kinases joue un rôle.

22. Utilisation selon la revendication 21, dans laquelle la kinase est la SGK.

23. Utilisation selon la revendication 22 de composés selon les revendications 1-18, et de sels, solvats et stéréoisomères pharmaceutiquement utilisables de ceux-ci, y compris des mélanges de ceux-ci en toutes proportions, pour la préparation d'un médicament destiné au traitement de maladies qui sont influencées par l'inhibition des SGK par les composés selon les revendications 1-18.

24. Utilisation selon la revendication 23 de composés selon les revendications 1-18, et de sels, solvats et stéréoisomères pharmaceutiquement utilisables de ceux-ci, y compris des mélanges de ceux-ci en toutes proportions, pour la préparation d'un médicament destiné au traitement ou à la prévention du diabète, de l'obésité, du syndrome métabolique (dyslipidémie), de l'hypertension systémique et pulmonaire, des maladies cardiovasculaires et des maladies rénales, généralement dans tous les types de fibroses et de processus inflammatoires, du cancer, des cellules tumorales, des métastases tumorales, des coagulopathies, de l'excitabilité neuronale, du glaucome, des cataractes, des infections bactériennes et de la thérapie anti-infectieuse, pour augmenter les capacités d'apprentissage et l'attention, et pour le traitement et la prophylaxie du stress et du vieillissement cellulaires, et pour le traitement de l'acouphène.

25. Utilisation selon la revendication 24, dans laquelle le diabète est le diabète sucré, la néphropathie diabétique, la neuropathie diabétique, l'angiopathie diabétique et la microangiopathie.

26. Utilisation selon la revendication 24, dans laquelle les maladies cardiovasculaires sont les fibroses cardiaques après un infarctus du myocarde, l'hypertrophie cardiaque, l'insuffisance cardiaque et l'artériosclérose.

27. Utilisation selon la revendication 24, dans laquelle les maladies rénales sont la glomérulosclérose, la néphrosclérose, la néphrite, la néphropathie et l'altération de l'excrétion des électrolytes.

28. Utilisation selon la revendication 24, dans laquelle les fibroses et les processus inflammatoires sont la cirrhose hépatique, la fibrose pulmonaire, la pancréatite fibrosante, les rhumatismes et les arthroses, la maladie de Crohn, la bronchite chronique, la fibrose induite par une radiothérapie, la sclérodermie, la mucoviscidose, la cicatrisation et la maladie d'Alzheimer.

29. Médicaments comprenant au moins un composé selon les revendications 1-18 et/ou des sels, solvats et stéréoisomères pharmaceutiquement utilisables de celui-ci, y compris des mélanges de ceux-ci en toutes proportions, et au moins un autre composé actif médicamenteux.

30. Ensemble (kit) constitué de conditionnements séparés
(a) d'une quantité efficace d'un composé selon les revendications 1-18 et/ou de sels, solvats et stéréoisomères pharmaceutiquement utilisables de celui-ci, y compris des mélanges de ceux-ci en toutes proportions,
et
(b) d'une quantité efficace d'un autre composé actif médicamenteux.
